# EUROPEAN PATENT APPLICATION

(11) **EP 4 473 979 A1**
(43) Date of publication of application: **11.12.2024**
(21) Application number: 23749928.0
(22) Date of filing: 27.01.2023
(51) Int. Cl.: A61K 49/00, A61K 47/64, A61K 47/61

(54) **NEAR-INFRARED FLUORESCENT DYE-LOADED POLYMER MICROSPHERES FOR LABELLING LESIONS, AND FORMULATION THEREOF**

(30) Priority: 04.02.2022 KR 20220014733; 08.07.2022 KR 20220084369
(71) Applicant: National Cancer Center, Goyang-si, Gyeonggi-do 10408 (KR)
(72) Inventor: CHOI, Yongdoo, Goyang-si, Gyeonggi-do 10408 (KR); KIM, Hyoung Jun, Goyang-si, Gyeonggi-do 10408 (KR)
(74) Representative: Algemeen Octrooi- en Merkenbureau B.V.
(86) International application number: PCT/KR2023/001265
(87) International publication number: WO 2023/149695

(57) **Abstract**

The present invention relates to near-infrared fluorescent dye-loaded microspheres and a formulation thereof, and specifically, to near-infrared fluorescent dye-loaded microspheres and a formulation thereof that, by injecting the microspheres into lesion sites such as cancer, can help enable precision surgery by accurately identifying the location of lesion sites from fluorescence imaging during surgery. The near-infrared fluorescent dye-loaded microspheres according to the present invention enable the location of lesion sites to be accurately determined in real time through fluorescence imaging when injected into lesion sites such as cancer, can dramatically increase the accuracy of surgery for lesion excision, and can significantly shorten the time required for surgery. By forming a complex of near-infrared fluorescent dye and human serum albumin or near-infrared fluorescent dye and cyclodextrin and loading it into microspheres, or by mixing the near-infrared fluorescent dye with a hydrogel polymer and loading it into microspheres, the intensity of fluorescence generated from the microspheres is stronger than that when microspheres are prepared using a near-infrared fluorescent dye alone, the stability of the fluorescent dye in the microspheres is improved, and the marked sites can be identified through fluorescent imaging for a long period of time.

## Description

### Technical Field

The present invention relates to near-infrared fluorescent dye-loaded microspheres and a formulation thereof, and specifically, to near-infrared fluorescent dye-loaded microspheres and a formulation thereof that, by injecting the microspheres into lesion sites such as cancer, can help enable precision surgery by accurately identifying the location of lesion sites from fluorescence imaging during surgery.

### Background Art

Since fluorescence imaging technology has the great advantage of being able to acquire real-time images with high sensitivity compared to magnetic resonance imaging, nuclear medicine imaging and ultrasound imaging, it is evaluated as the most powerful tool for identifying and removing the location of lesions such as cancer in real time during surgery. Moreover, as systems capable of implementing near-infrared fluorescence imaging functions in endoscopic and laparoscopic equipment have recently become increasingly commercialized, procedures using fluorescence imaging are expected to be widely used in patient procedures in the future.

Laparoscopic surgery is one of the "minimally invasive surgical" methods that reduces injury or impact on the patient's body and is a method of performing surgery using surgical tools (forceps, electric scalpels, hemostatic suturing device, and the like) while viewing the abdomen by creating a small hole of 0.5 to 1.5 cm in size instead of making an incision in the abdomen or chest and inserting an endoscope (laparoscope) equipped with a special camera. Laparoscopic surgery has been developed over the past several decades due to its advantages, such as less postoperative pain compared to laparotomy, faster recovery due to a smaller incision area, and shorter hospitalization period. Recently, with the introduction of robotic surgery, and the like, to which laparoscopic surgery is applied, laparoscopic surgery has been accepted as the basis of surgical maneuvers. For surgical treatment of colorectal cancer, stomach cancer, and the like, it is necessary to insert an endoscope into the stomach or large intestine to visually identify procedure sites, for example, cancerous sites, before incising and removing cancerous sites in the body through laparoscopic surgery. However, because the viewing angle and range obtained through an endoscope in the conventional procedure are completely different from those obtained through a laparoscope, it is difficult to accurately find the target location for incision, and thus, the accuracy and range of the incision are bound to differ depending on the operator's skill level, and the range of the incision is also bound to increase. Thus, in order to mark the sites to be removed by surgery, a method is being used in which a drug such as Indian ink is injected into the lesion identified through an endoscope through an endoscopic injection needle to mark the lesion sites, and then surgery is performed while visually identifying the sites marked with Indian ink through a laparoscope during surgery. However, this marking method has the disadvantage that complications occur due to ink incorrectly injected into the muscle layer, and the sites to be removed by surgery widen as the ink or dye spreads from the injected sites to surrounding sites. Thus, in order to minimize the sites where normal tissue is removed during laparoscopic surgery, a contrast agent for "marking" or "marker" is needed to indicate the appropriate location of the disease lesion.

Recently, attempts have been made to use near-infrared fluorescent dyes to mark lesions. Fluorescence imaging technology using near-infrared fluorescent dyes has the advantage of not only being able to detect marked sites located deep in tissues from fluorescence imaging, but also being able to sensitively detect fluorescence signals even when using a small amount of fluorescent dye. Since fluorescence imaging technology has the great advantage of being able to acquire real-time images with high sensitivity compared to magnetic resonance imaging, nuclear medicine imaging and ultrasound imaging, it is evaluated as the most powerful tool for identifying and removing the location of lesions such as cancer in real time during surgery. As a fluorescent imaging contrast agent that has been attempted previously, indocyanine green, which is a near-infrared fluorescent dye approved for clinical use by the U.S. Food and Drug Administration (FDA), has been used in vascular imaging, and the like, but has several limitations in clinical application. Indocyanine green has reduced fluorescence brightness due to concentration-dependent aggregation phenomenon and is prone to decomposition due to chemical instability in aqueous solutions. In addition, because it spreads or is absorbed quickly from the injected sites to surrounding tissues, it has the disadvantage that fluorescence signals are generated in a wider range than the injected sites or that the fluorescence signals weaken within a short period of time after injection. For this reason, there is a need to develop a method that increases the efficiency of marking lesions and maintains high fluorescence signals of near-infrared fluorescent dye for a long time without fluorescence blurring in the marked sites.

Long Wang et al. prepared indocyanine green dye-loaded microbubbles for detection of sentinel lymph nodes using ultrasound imaging and fluorescence imaging. Microbubbles made of poly(D,L-lactide-co-glycolide) (PLGA), which is a biodegradable polymer, exhibit an ultrasound imaging contrast effect, and by loading indocyanine green into the microbubbles, when the microbubbles migrated from the injected site and accumulated in the sentinel lymph nodes, the location of the sentinel lymph nodes could be identified from ultrasound imaging and near-infrared fluorescence imaging. However, as mentioned above, since indocyanine green tends to aggregate with each other in aqueous solution and is unstable, if indocyanine green is used alone and loaded into microbubbles, there is a problem that the intensity of fluoresce signal is greatly weakened by the aggregation phenomenon of indocyanine green molecules, and the fluorescence intensity is further reduced by the decomposition of indocyanine green over time. In addition, in the case of microbubbles, if a hole is created in the shell layer during the preparation process or by *in vivo* hydrolysis, as the loaded indocyanine green is quickly released to the outside, a decrease in fluorescence signals and a blurring phenomenon of fluorescence signals to surrounding tissues may occur.

The present invention has been devised to solve the problems of the prior art as described above, and the present inventors have developed polymer microspheres loaded with a near-infrared fluorescent dye complex consisting of a near-infrared fluorescent dye and human serum albumin, or a near-infrared fluorescent dye and cyclodextrin and completed the present invention. Since near-infrared fluorescent dye-loaded microspheres allow the fluorescent dye loaded within the microspheres to remain within the microspheres for a long period of time while generating a strong fluorescence signal, it is possible to accurately and over a long period of time determine the location of the lesion into which the microspheres were injected through fluorescence imaging.

If the location of lesions is marked using the near-infrared fluorescent dye-loaded microspheres and their formulation developed in the present invention, it is possible to accurately excise lesions by surgery while identifying the marked location through fluorescence imaging, and to dramatically shorten the surgical time. In addition, since the near-infrared fluorescent dye loaded in the microspheres may be tracked through imaging for a long period of time, it is also possible to use them to track changes that occur at the marked location over a long period of time.

### Disclosure

### Technical Problem

It is an exemplary object of the present invention to provide near-infrared fluorescent dye-loaded polymer microspheres for marking lesions, wherein the near-infrared fluorescent dye forms a complex with human serum albumin or cyclodextrin.

It is another exemplary object of the present invention to provide a method for preparing the polymer microspheres.

It is another exemplary object of the present invention to provide a composition for marking lesions, comprising the polymer microspheres.

It is another exemplary object of the present invention to provide a method for marking lesions, comprising the step of injecting the composition into an individual.

It is another exemplary object of the present invention to provide use of the composition for marking lesions.

The technical problems to be achieved according to the technical idea of the invention disclosed in the present specification are not limited to the above-mentioned problems to be solved, and other problems not mentioned will be clearly understood by those skilled in the art from the description below.

### Technical Solution

These will be described in detail as follows. On the other hand, each description and embodiment disclosed in the present application may also be applied to each other description and embodiment. That is, all combinations of the various elements disclosed in this application fall within the scope of this application. In addition, it cannot be seen that the scope of the present application is limited by the specific descriptions described below.

As an aspect for achieving the above objects, the present invention provides near-infrared fluorescent dye-loaded polymer microspheres for marking lesions, wherein the near-infrared fluorescent dye forms a complex with human serum albumin or cyclodextrin.

The marking of the lesions allows accurate surgery and shortened surgery time by marking the location of the lesions to be removed by surgery in advance before surgery and identifying the marked location in real time through fluorescence imaging during surgery.

In the present invention, the near-infrared fluorescent dye is any one selected from, but is not limited to, indocyanine green, IRDye 800CW carboxylate (IRDye 800CW), Alexa Fluor 647, Alexa Fluor 660, Alexa Fluor 680, Alexa Fluor 700, Alexa Fluor 750, Alexa Fluor 780, Flamma 749, Flamma 774, Flamma 800, FSD Fluor^{™} 647, FSD Fluor^{™} 680, FSD Fluor^{™} 750, FSD Fluor^{™} 800, sulfo-cyanine 5 carboxylic acid (Cy5), sulfo-cyanine 5.5 carboxylic acid (Cy5.5), sulfo-cyanine 7 carboxylic acid (Cy7) or sulfo-cyanine 7.5 carboxylic acid (Cy7.5).

The near-infrared fluorescent dye may be a dye that generates a fluorescence signal at a wavelength of 650 nm or more.

The indocyanine green is a fluorescent imaging contrast agent approved for clinical use by the U.S. Food and Drug Administration (FDA) and has been used in vascular imaging, and the like, but has several limitations in clinical application. Indocyanine green has reduced fluorescence brightness due to concentration-dependent aggregation and is prone to decomposition due to chemical instability in aqueous solutions. In addition, because it spreads or is absorbed quickly from the injected sites to surrounding tissues, it has the disadvantage that fluorescence signals are generated in a wider range than the injected sites or that the fluorescence signals weaken within a short period of time after injection. For this reason, there is a need to develop a method that increases the efficiency of marking lesions and maintains high fluorescence signals of near-infrared fluorescent dye for a long time without fluorescence blurring in the marked sites.

Since indocyanine green tends to aggregate with each other in aqueous solution and is unstable, if indocyanine green is used alone, there is a problem that the intensity of fluoresce signal is greatly weakened by the aggregation phenomenon of indocyanine green molecules, and the fluorescence intensity is further reduced by the decomposition of indocyanine green over time.

Since the microspheres loaded with a complex of a near-infrared fluorescent dye and human serum albumin, or a near-infrared fluorescent dye and cyclodextrin according to the present invention allow the fluorescent dye loaded within the microspheres to remain within the microspheres for a long period of time while generating a strong fluorescence signal, it is possible to accurately and over a long period of time determine the location of the target sites through fluorescence imaging.

In the present invention, a water-in-oil-in-water (W1/O/W2) emulsion method may be used as a method for loading the complex into the polymer microspheres. If a near-infrared fluorescent dye with low water solubility is loaded alone into the microspheres, there is a disadvantage that as aggregation of the near-infrared fluorescent dyes occurs within the microspheres, the intensity of the fluorescence signal generated from the prepared microspheres becomes very weak, and the chemical/physical stability of the near-infrared fluorescent dye is also lowered. In the case of forming a complex of a near-infrared fluorescent dye and human serum albumin, or a near-infrared fluorescent dye and cyclodextrin and loading it into microspheres, the aggregation phenomenon that occurs when the fluorescent dye is loaded alone may be prevented, the fluorescence signal generated from the microspheres is also much stronger, and the chemical/physical stability of the fluorescent dye is also increased.

In the present invention, the polymer is a biodegradable polymer and may be, but is not limited to, poly(lactide-co-glycolide) (PLGA), poly(DL-lactide-co-glycolide) (PDLGA), poly(glycolic acid) (PGA), poly(lactide) (PLA), poly(hydroxybutyrate), polycaprolactone (PCL), polydioxanone (PDO), poly(amino acid), polyanhydride, polyorthoester or polyphosphazene.

In the present invention, the polymer may form a block copolymer with poly(ethylene oxide) (PEG).

As an example, the polymer may be one that forms a block copolymer with poly(D,L-lactide)-block-poly(ethylene glycol) (PDLLA-PEG) .

In the present invention, the polymer may be poly(methyl methacrylate) (PMMA) or polycarbonate (PC), which are non-biodegradable polymers.

In the present invention, the microspheres may further comprise alginic acid or hyaluronic acid along with a complex of a near-infrared fluorescent dye and human serum albumin, or a near-infrared fluorescent dye and cyclodextrin. When the complex is dispersed in an alginic acid or hyaluronic acid hydrogel and loaded, the aggregation phenomenon between fluorescent dyes may be prevented better compared to the case where the complex is loaded alone in the microspheres, the fluorescence signal generated from the microspheres becomes remarkably stronger, and the chemical and physical stability of the fluorescent dyes is also increased.

In addition, if a near-infrared fluorescent dye, a near-infrared fluorescent dye and human serum albumin, or a near-infrared fluorescent dye and cyclodextrin, present in W1 phase are mixed with hydrogel using polymers such as alginic acid or hyaluronic acid, the rate at which the fluorescent dye is released out of the microspheres may be further slowed.

In the present invention, the microspheres comprise one or more cavities (isolated spaces) within the microspheres, the complex of the near-infrared fluorescent dye and human serum albumin, or the near-infrared fluorescent dye and cyclodextrin may be loaded into the cavities.

In particular, if several isolated spaces are created within the microspheres and the near-infrared fluorescent dye is loaded thereinto, even if the outer shell of the microspheres decomposes and creates holes, rapid release of the fluorescent dye loaded in the isolated spaces within the microspheres does not occur. In this way, by creating several isolated spaces within the microspheres and loading fluorescent dye thereinto, the problem of unwanted release of fluorescent dye from microbubbles may be solved.

In the present invention, the microspheres may further comprise a surfactant.

The surfactant may be any one selected from polyvinyl alcohol, polyethylene glycol, Labrafil, Labrasol, medium chain triglyceride, lecitin, N-methyl pyrrolidone, polyvinyl pyrrolidone, hydropropyl methylcellulose, poloxamer (e.g., poloxamer 188 (P-188), poloxamer 407 (P-407)) or Tween (e.g., Tween-80, Tween-20).

The microspheres of the present invention may be dispersed in an aqueous solution in which the surfactant or hydrophilic polymer is dissolved for injection into the marked sites. Types of the hydrophilic polymer include carboxymethylcellulose, gelatin, collagen, alginic acid and hyaluronic acid.

If the microspheres are prepared in the form of pellets together with a hydrogel polymer and injected using a syringe into the sites to be marked, it is possible to prevent migration of the microspheres from the injected sites and further inhibit the release of fluorescent dye into surrounding tissues. The hydrogel polymer may be a temperature-sensitive polymer such as poloxamer, or may be alginic acid, hyaluronic acid or derivatives thereof that cause an ionic crosslinking reaction.

In the present invention, the microspheres may be for coating on the surface of a metal or non-metallic material.

The coating may be made with adhesives or rubber.

In an embodiment of the present invention, a lesion marker may be prepared using a metal and/or non-metal material by coating the microspheres with adhesives or rubber on the surface of the metal and/or non-metal material.

The adhesives may be, but is not limited to, for example, urea-based, melamine-based, phenol-based, unsaturated polyester-based, epoxy-based, resorcinol-based, vinyl acetate-based, polyvinyl alcohol-based, vinyl chloride-based, polyvinyl acetal-based, acrylic-based, saturated polyester-based, polyamide-based or polyethylene-based.

The rubber may be, but is not limited to, for example, butadiene rubber-based, nitrile rubber-based, butyl rubber-based, silicone rubber-based, chloroprene rubber-based, latex-free rubber (NFR), neoprene, natural latex or synthetic latex.

As another aspect for achieving the above objects, the present invention provides a method for preparing polymer microspheres for marking lesions, comprising the steps of: forming a complex of a near-infrared fluorescent dye and human serum albumin, or a near-infrared fluorescent dye and cyclodextrin; and loading the complex into polymer microspheres.

In the present invention, a water-in-oil-in-water (W1/O/W2) emulsion method may be used as a method for loading the complex into the polymer microspheres.

In the present invention, the method may further comprise mixing the near-infrared fluorescent dye with a hydrogel polymer to load it into the polymer microspheres.

In the present invention, the microspheres may be prepared by mixing a polymer and a surfactant in oil phase for the purpose of further increasing the fluorescence signal of the near-infrared fluorescent dye loaded in the microspheres.

As another aspect for achieving the above objects, the present invention provides a composition for marking lesions, comprising the polymer microspheres.

The fluorescent dye, polymer, microspheres and surfactant are as described above.

In the present invention, the microspheres may be mixed with methylcellulose, alginic acid, hyaluronic acid, polyvinyl alcohol, collagen, gelatin, Tween, poloxamer or polyvinyl pyrrolidone. Specifically, the microspheres may be in the form dispersed in an aqueous solution in which methylcellulose, alginic acid, hyaluronic acid, polyvinyl alcohol, collagen, gelatin, Tween, poloxamer or polyvinylpyrrolidone is dissolved.

In the present invention, the composition for marking the lesions may be prepared so that it is injectable through a syringe.

In the present invention, the composition for marking the lesions may be in the form of a liquid or solid pellet, and specifically may be in the form of a solid pellet.

As another aspect for achieving the above objects, the present invention provides a method for marking lesions, comprising the step of injecting the composition into an individual.

In an embodiment of the present invention, the individual is not particularly limited, but includes, for example, a human, a monkey, a cow, a horse, a sheep, a pig, a chicken, a turkey, a quail, a cat, a dog, a mouse, a rat, a rabbit or a guinea pig, and may be specifically a mammal, more specifically a human.

As another aspect for achieving the above objects, the present invention provides use of the composition for marking lesions.

### Advantageous Effects

The near-infrared fluorescent dye-loaded microspheres according to the present invention enable the location of lesion sites to be accurately determined in real time through fluorescence imaging when injected into lesion sites such as cancer, can dramatically increase the accuracy of surgery for lesion excision, and can significantly shorten the time required for surgery. By forming a complex of near-infrared fluorescent dye and human serum albumin or near-infrared fluorescent dye and cyclodextrin and loading it into microspheres, or by mixing the near-infrared fluorescent dye with a hydrogel polymer and loading it into microspheres, the intensity of fluorescence generated from the microspheres is stronger than that when microspheres are prepared using a near-infrared fluorescent dye alone, the stability of the fluorescent dye in the microspheres is improved, and the marked sites can be identified through fluorescent imaging for a long period of time.

### Description of Drawings

Figure 1 shows the composition for the shape and formulation of a near-infrared fluorescent dye complex-loaded polymer microspheres.
Figure 2 shows fluorescence spectrum data obtained by dissolving the same concentration of indocyanine green (ICG) and indocyanine green-human serum albumin (ICG-HSA) complex in an aqueous solution. It can be seen that ICG shows a higher fluorescence signal when complexed with HSA.
Figure 3 shows scanning electron microscopy images of (a) ICG-PLGA (without P-188), (b) ICG-HSA-PLGA (without P-188) and (c) ICG-HSA-PLGA (P-188 0.1%) microspheres.
Figure 4 shows a fluorescence spectrum for comparison of fluorescence signals of ICG-HSA solution, and dispersions of ICG-PLGA (without P-188), ICG-HSA-PLGA (without P-188) and ICG-HSA-PLGA (P-188 0.1%) microspheres. All samples were adjusted to a concentration of 30 µM equivalent based on ICG. In the case of a dispersion of ICG-PLGA (without P-188) microspheres loaded with ICG alone, the fluorescence signal is 6.8 times lower than that of ICG-HSA, and it can be seen that when the ICG-HSA complex is loaded, microspheres generating a higher fluorescence signal may be prepared.
Figure 5 shows scanning electron microscopy images of (a) the surface of ICG-HSA-PLGA (P-188 0.5%) microspheres, (b) the cross section of ICG-HSA-PLGA (P-188 0.5%) microspheres, (c) the surface of ICG-HSA-PLGA (P-188 1.0%) microspheres and (d) the cross section of ICG-HSA-PLGA (P-188 1.0%) microspheres.
Figure 6 shows a fluorescence spectrum for comparison of fluorescence signals of ICG-HSA solution, and dispersions of ICG-PLGA (without P-188), ICG-HSA-PLGA (P-188 0.5%) and ICG-HSA-PLGA (P-188 1.0%) microspheres.
Figure 7 shows fluorescence images of (a) ICG-HSA and dispersions of (b) ICG-HSA-PLGA (P-188 0.5%) microspheres and (c) ICG-HSA-PLGA (P-188 1.0%) microspheres (λₑₓ = 780/20 nm, λₑₘ = 845/40 nm).
Figure 8 shows fluorescence images taken over time after subcutaneously injecting (a) ICG-HSA and dispersions of (b) ICG-HSA-PLGA (P-188 0.5%) microspheres and (c) ICG-HSA-PLGA (P-188 1.0%) microspheres into SKH-1 hairless mice (λₑₓ = 780/20 nm, λₑₘ = 845/40 nm).
Figure 9 shows a graph analyzing ROI (region of interest) values of fluorescence signals at the injected sites over time after subcutaneously injecting ICG-HSA, and dispersions of ICG-HSA-PLGA (P-188 0.5%) microspheres and ICG-HSA-PLGA (P-188 1.0%) microspheres into SKH-1 hairless mice.
Figure 10 shows scanning electron microscopy images of the (a) surface and (b) cross section of ICG-HSA-Alginate-PLGA, PDLLA-PEG (P-188 0.1%) microspheres.
Figure 11 shows a fluorescence spectrum for comparison of fluorescence signals of ICG-HSA solution, and dispersions of ICG-PLGA (without P-188) and ICG-HSA-Alginate-PLGA, PDLLA-PEG (P-188 0.1%) microspheres.
Figure 12 shows fluorescence images of (a) ICG-HSA and (b) a dispersion of ICG-HSA-Alginate-PLGA, PDLLA-PEG (P-188 0.1%) microspheres (λₑₓ = 780/20 nm, λₑₘ = 845/40 nm) .
Figure 13 shows fluorescence images taken over time after subcutaneously injecting (a) ICG-HSA and (b) a dispersion of ICG-HSA-Alginate-PLGA, PDLLA-PEG (P-188 0.1%) microspheres into SKH-1 hairless mice (λₑₓ = 780/20 nm, λₑₘ = 845/40 nm) .
Figure 14 shows a graph analyzing ROI (region of interest) values of fluorescence signals at the injected sites over time after subcutaneously injecting ICG-HSA and a dispersion of ICG-HSA-Alginate-PLGA, PDLLA-PEG (P-188 0.1%) microspheres into SKH-1 hairless mice.
Figure 15 shows scanning electron microscopy images of the (a) surface and (b) cross section of ICG-HSA-PMMA (P-188 1.0%) microspheres.
Figure 16 shows a fluorescence spectrum for comparison of fluorescence signals of ICG-HSA solution and dispersions of ICG-PLGA (without P-188) and ICG-HSA-PMMA (P-188 1.0%) microspheres.
Figure 17 shows fluorescence images of (a) ICG-HSA and (b) a dispersion of ICG-HSA-PMMA (P-188 1.0%) microspheres (λₑₓ = 780/20 nm, λₑₘ = 845/40 nm) .
Figure 18 shows fluorescence images taken over time after subcutaneously injecting (a) ICG-HSA and (b) a dispersion of ICG-HSA-PMMA (P-188 1.0%) microspheres into SKH-1 hairless mice (λₑₓ = 780/20 nm, λₑₘ = 845/40 nm) .
Figure 19 shows a graph analyzing ROI (region of interest) values of fluorescence signals at the injected sites over time after subcutaneously injecting ICG-HSA and a dispersion of ICG-HSA-PMMA (P-188 1.0%) microspheres into SKH-1 hairless mice.
Figure 20 shows scanning electron microscopy images of (a) ICG-Alginate-PLGA (without P-188) and (b) ICG-Alginate-PLGA (P-188 0.1%) microspheres.
Figure 21 shows a fluorescence spectrum for comparison of fluorescence signals of ICG-HSA solution, and dispersions of ICG-PLGA (without P-188), ICG-Alginate-PLGA (without P-188) and ICG-Alginate-PLGA (P-188 1.0%) microspheres.
Figure 22 shows scanning electron microscopy images of (a) the surface of ICG-HSA-Alginate-PLGA (P-188 0.1%) microspheres, (b) the surface of ICG-HSA-Alginate-PLGA (P-188 0.5%) microspheres and (c) the cross section of ICG-HSA-Alginate-PLGA (P-188 0.5%) microspheres.
Figure 23 shows a fluorescence spectrum for comparison of fluorescence signals of ICG-HSA solution, and dispersions of ICG-PLGA (without P-188), ICG-HSA-Alginate-PLGA (P-188 0.1%) and ICG-HSA-Alginate-PLGA (P-188 0.5%) microspheres.
Figure 24 shows fluorescence images of (a) ICG-HSA and dispersions of (b) ICG-HSA-Alginate-PLGA (P-188 0.1%) and (c) ICG-HSA-Alginate-PLGA (P-188 0.5%) microspheres (λₑₓ = 780/20 nm, λₑₘ = 845/40 nm).
Figure 25 shows fluorescence images taken over time after subcutaneously injecting (a) ICG-HSA and (b) a dispersion of ICG-HSA-Alginate-PLGA (P-188 0.5%) microspheres into SKH-1 hairless mice (λₑₓ = 780/20 nm, λₑₘ = 845/40 nm) .
Figure 26 shows a graph analyzing ROI (region of interest) values of fluorescence signals at the injected sites over time after subcutaneously injecting ICG-HSA and a dispersion of ICG-HSA-Alginate-PLGA (P-188 0.5%) microspheres into SKH-1 hairless mice.
Figure 27 shows scanning electron microscopy images of (a) the surface of (a) the surface of ICG-HSA-Alginate-PLGA (Tween 0.5%) microspheres and (b) the cross section of ICG-HSA-Alginate-PLGA (Tween 0.5%) microspheres.
Figure 28 shows a fluorescence spectrum for comparison of fluorescence signals of ICG-HSA solution and dispersions of ICG-PLGA (without P-188) and ICG-HSA-Alginate-PLGA (Tween 0.5%) microspheres.
Figure 29 shows fluorescence images of (a) ICG-HSA and (b) a dispersion of ICG-HSA-Alginate-PLGA (Tween 0.5%) microspheres (λₑₓ = 780/20 nm, λₑₘ = 845/40 nm) .
Figure 30 shows fluorescence images taken over time after subcutaneously injecting (a) ICG-HSA and (b) a dispersion of ICG-HSA-Alginate-PLGA (Tween 0.5%) microspheres into SKH-1 hairless mice (λₑₓ = 780/20 nm, λₑₘ = 845/40 nm) .
Figure 31 shows a graph analyzing ROI (region of interest) values of fluorescence signals at the injected sites over time after subcutaneously injecting ICG-HSA and a dispersion of ICG-HSA-Alginate-PLGA (Tween 0.5%) microspheres into SKH-1 hairless mice.
Figure 32 shows scanning electron microscopy images of (a) the surface of ICG-mCD-Alginate-PLGA (P-188 0.1%) microspheres and (b) the cross section of ICG-mCD-Alginate-PLGA (P-188 0.1%) microspheres.
Figure 33 shows fluorescence spectra for comparison of fluorescence signals of (a) ICG solution and ICG-mCD and (b) ICG-HSA solution and dispersions of ICG-PLGA (without P-188) and ICG-mCD-Alginate-PLGA (P-188 0.1%) microspheres.
Figure 34 shows fluorescence images of (a) ICG-HSA and (b) a dispersion of ICG-mCD-Alginate-PLGA (P-188 0.1%) microspheres (λₑₓ = 780/20 nm, λₑₘ = 845/40 nm) .
Figure 35 shows fluorescence images taken over time after subcutaneously injecting (a) ICG-HSA and (b) a dispersion of ICG-mCD-Alginate-PLGA (P-188 0.1%) microspheres into SKH-1 hairless mice (λₑₓ = 780/20 nm, λₑₘ = 845/40 nm) .
Figure 36 shows a graph analyzing ROI (region of interest) values of fluorescence signals at the injected sites over time after subcutaneously injecting ICG-HSA and a dispersion of ICG-mCD-Alginate-PLGA (P-188 0.5%) microspheres into SKH-1 hairless mice.
Figure 37 shows a graph comparing fluorescence signal intensity depending on concentration of Cy7 and Cy7-HSA solutions (λₑₓ = 730 nm, λₑₘ = 780 nm (Cy7), λₑₘ = 786 nm (Cy7-HSA)).
Figure 38 shows scanning electron microscopy images of (a) the surface of Cy7-PMMA (without P-188) microspheres, (b) the cross section of Cy7-PMMA (without P-188) microspheres, (c) the surface of Cy7-HSA-PMMA (without P-188) microspheres, (d) the cross section of Cy7-HSA-PMMA (without P-188) microspheres, (e) the surface of Cy7-HSA-PMMA (P-188 1.0%) microspheres and (f) the cross section of Cy7-HSA-PMMA (P-188 1.0%) microspheres.
Figure 39 shows a graph comparing the fluorescence spectra of aqueous Cy7 solution, aqueous Cy7-HSA solution, a dispersion of Cy7-PMMA (without P-188) microspheres, a dispersion of Cy7-HSA-PMMA (without P-188) microspheres and a dispersion of Cy7-HSA-PMMA (P-188 1.0%) microspheres prepared at the same Cy7 concentration (30 µM) (λₑₓ = 730 nm) .
Figure 40 shows a graph comparing fluorescence signal intensity depending on concentration of Cy7.5 and Cy7.5-HSA solutions (λₑₓ = 730 nm, λₑₘ = 810 nm (Cy7.5), λₑₘ = 824 nm (Cy7.5-HSA)).
Figure 41 shows scanning electron microscopy images of (a) the surface of Cy7.5-PMMA (without P-188) microspheres, (b) the cross section of Cy7.5-PMMA (without P-188) microspheres, (c) the surface of Cy7.5-HSA-PMMA (without P-188) microspheres, (d) the cross section of Cy7.5-HSA-PMMA (without P-188) microspheres, (e) the surface of Cy7.5-HSA-PMMA (P-188 1.0%) microspheres and (f) the cross section of Cy7.5-HSA-PMMA (P-188 1.0%) microspheres.
Figure 42 shows a graph comparing the fluorescence spectra of aqueous Cy7.5 solution, aqueous Cy7.5-HSA solution, a dispersion of Cy7.5-PMMA (without P-188) microspheres, a dispersion of Cy7.5-HSA-PMMA (without P-188) microspheres and a dispersion of Cy7.5-HSA-PMMA (P-188 1.0%) microspheres prepared at the same Cy7.5 concentration (30 µM) (λₑₓ = 730 nm).
Figure 43 shows scanning electron microscopy images of (a) the surface of Cy7.5-PC (without P-188) microspheres, (b) the cross section of Cy7.5-PC (without P-188) microspheres, (c) the surface of Cy7.5-HSA-PC (without P-188) microspheres and (d) the cross section of Cy7.5-HSA-PC (without P-188) microspheres.
Figure 44 shows a graph comparing the fluorescence spectra of dispersions of Cy7.5-PC (without P-188) and Cy7.5-HSA-PC (without P-188) microspheres prepared at the same Cy7.5 concentration (λₑₓ = 730 nm).
Figure 45 shows a graph comparing fluorescence signal intensity depending on concentration of Flamma774 and Flamma774-HSA solutions (Δₑₓ = 730 nm, λₑₘ = 812 nm (Flamma774), λₑₘ = 818 nm (Flamma774-HSA)) .
Figure 46 shows scanning electron microscopy images of (a) the surface of Flamma774-PMMA (without P-188) microspheres, (b) the cross section of Flamma774-PMMA (without P-188) microspheres, (c) the surface of Flamma774-HSA-PMMA (without P-188) microspheres, (d) the cross section of Flamma774-HSA-PMMA (without P-188) microspheres, (e) the surface of Flamma774-HSA-PMMA (P-188 1.0%) microspheres and (f) the cross section of Flamma774-HSA-PMMA (P-188 1.0%) microspheres.
Figure 47 shows a fluorescence spectrum for comparison of fluorescence signals of Flamma774 solution, Flamma774-HSA solution, a dispersion of Flamma774-PMMA (without P-188) microspheres, a dispersion of Flamma774-HSA-PMMA (without P-188) microspheres and a dispersion of Flamma774-HSA-PMMA (P-188 1.0%) microspheres.
Figure 48 shows a graph comparing fluorescence signal intensity depending on concentration of Alexa750 and Alexa750-HSA solutions (Δₑₓ = 730 nm, λₑₘ = 776 nm) .
Figure 49 shows scanning electron microscopy images of cross sections of (a) Alexa750-PMMA (without P-188) microspheres, (b) Alexa750-HSA-PMMA (without P-188) microspheres and (c) Alexa750-HSA-PMMA (P-188 1.0%) microspheres.
Figure 50 shows a fluorescence spectrum for comparison of fluorescence signals of Alexa750 solution, Alexa750-HSA solution, a dispersion of Alexa750-PMMA (without P-188) microspheres, a dispersion of Alexa750-HSA-PMMA (without P-188) microspheres and a dispersion of Alexa750-HSA-PMMA (P-188 1.0%) microspheres.
Figure 51 shows a graph comparing fluorescence signal intensity depending on concentration of IRDye 800CW and IRDye 800CW-HSA solutions (λₑₓ = 730 nm, λₑₘ = 806 nm (IRDye 800CW), λₑₘ = 810 nm (IRDye 800CW-HSA)).
Figure 52 shows scanning electron microscopy images of (a) the surface of IRDye 800CW-PMMA (without P-188) microspheres, (b) the cross section of IRDye 800CW-PMMA (without P-188) microspheres, (c) the surface of IRDye 800CW-HSA-PMMA (without P-188) microspheres, (d) the cross section of IRDye 800CW-HSA-PMMA (without P-188) microspheres, (e) the surface of IRDye 800CW-HSA-PMMA (P-188 1.0%) microspheres and (f) the cross section of IRDye 800CW-HSA-PMMA (P-188 1.0%) microspheres.
Figure 53 shows a fluorescence spectrum for comparison of fluorescence signals of IRDye 800CW solution, IRDye 800CW-HSA solution, a dispersion of IRDye 800CW-PMMA (without P-188) microspheres, a dispersion of IRDye 800CW-HSA-PMMA (without P-188) microspheres and a dispersion of IRDye 800CW-HSA-PMMA (P-188 1.0%) microspheres.
Figure 54 shows photographs taken after dispersing ICG-HSA-PLGA (P-188 0.5%) microspheres in aqueous solutions of 0.5% sodium methylcellulose/0.1% Tween 80, 1% sodium alginate, 1% sodium hyaluronate, 2% sodium methylcellulose, 2% poly(vinyl alcohol), 0.1% collagen, 5% gelatin, 10% Tween 80, 40% poloxamer 188 and 5% polyvinyl pyrrolidone to prepare a composition in which the microspheres were dispersed in a surfactant or polymer solution.
Figure 55 shows photographs of cylindrical pellets comprising the microspheres, prepared by dispersing ICG-HSA-PLGA (P-188 0.5%) microspheres in aqueous solutions of 1% sodium hyaluronate and 5% gelatin and lyophilizing them, respectively.
Figure 56 shows photographs taken after dispersing ICG-HSA-PMMA (P-188 1.0%) microspheres in aqueous solutions of 0.5% sodium methylcellulose/0.1% Tween 80, 1% sodium alginate, 1% sodium hyaluronate, 2% sodium methylcellulose, 2% poly(vinyl alcohol), 0.1% collagen, 5% gelatin, 10% Tween 80, 40% poloxamer 188 (P-188) and 5% polyvinyl pyrrolidone to prepare a composition in which the microspheres were dispersed in a surfactant or polymer solution.
Figure 57 shows photographs of cylindrical pellets comprising the microspheres, prepared by dispersing in aqueous solutions of 1% sodium alginate, 1% sodium hyaluronate, 2% sodium methylcellulose, 2% poly(vinyl alcohol), 5% gelatin, 40% poloxamer 188 and 5% polyvinyl pyrrolidone and lyophilizing them.
Figure 58 shows photographs of a metal clip coated with fluorescent microspheres, prepared by dispersing IRDye 800CW-HSA-PMMA (without P-188) microspheres in a latex solution, followed by coating on the surface of the metal clip and drying.
Figure 59 shows a near-infrared fluorescence image of a metal clip coated with IRDye 800CW-HSA-PMMA (without P-188) microspheres (λₑₓ = 780/20 nm, λₑₘ = 845/40 nm) .

### Best Mode

Hereinafter, the present invention will be described in more detail through the following examples. However, these examples are only for illustrating the present invention, and the scope of the present invention is not limited to these examples only.

### Example 1: Comparison of fluorescence signals of indocyanine green solution and indocyanine green/human serum albumin complex

0.5 mg of indocyanine green (Dianogreen Injection) (ICG, Jeil Pharmaceutical Co., Ltd.) as a near-infrared fluorescent dye was dissolved in 1 mL of distilled water, and then diluted with distilled water to prepare an indocyanine green solution at a concentration of 30 µM. For the indocyanine green/human serum albumin (HSA, SK Plasma) complex for comparison of fluorescence signals with the indocyanine green solution, 0.5 mg of indocyanine green powder was dissolved in a mixed solution of 0.214 mL of human serum albumin solution (20% concentration) and 0.786 mL of distilled water, and then diluted with distilled water to prepare an indocyanine green/human serum albumin complex (ICG-HSA) at a concentration of 30 µM. To compare fluorescence spectra, 200 µL of each prepared solution was aliquoted and placed in a 96-well plate, and the fluorescence spectrum (λₑₓ = 730 nm) was measured using a multi-function microplate reader (SPARK, Tecan Trading AG, Zurich, Switzerland).

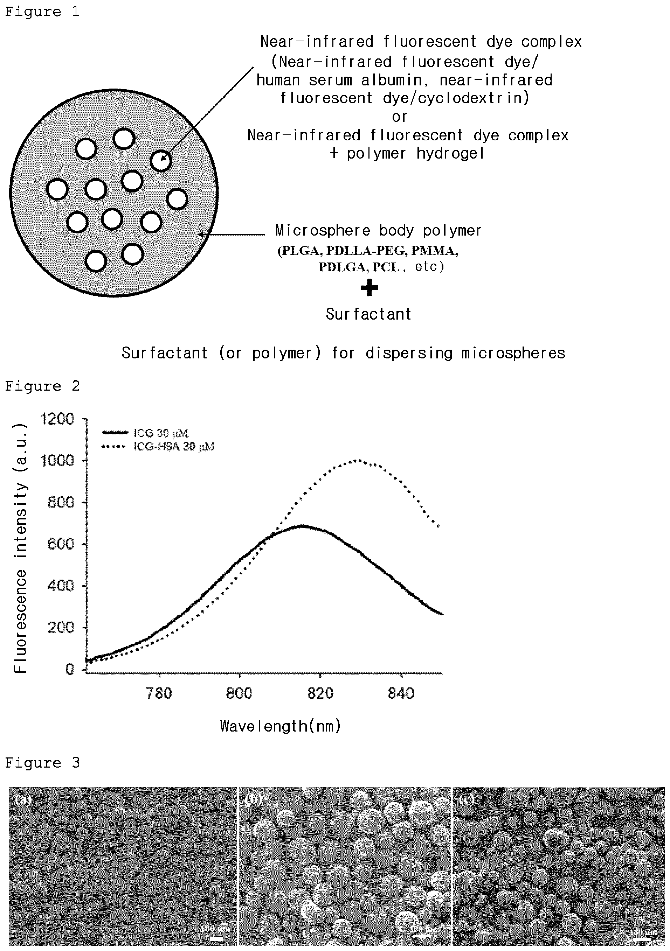

As a result of measuring the fluorescence spectra of the same concentration of indocyanine green solution and indocyanine green/human serum albumin complex solution, as shown in Figure 2, it could be confirmed that the indocyanine green/human serum albumin complex generated a fluorescence signal 1.5 times higher than that of indocyanine green.

### Example 2: Preparation and analysis of PLGA microspheres loaded with ICG or ICG-HSA

### 2-1. Preparation of ICG-loaded PLGA microspheres

ICG-PLGA (without P-188), which is PLGA microspheres loaded with indocyanine green alone as a near-infrared fluorescent dye, was prepared by the water-in-oil-in-water (W1/O/W2) emulsion method.

Specifically, 0.75 mg of indocyanine green (ICG, Selleck chem) was added to 1.5 mL of distilled water and then dissolved to prepare W1 solution. 3.6 g of poly(vinyl alcohol) (PVA, Sigma-Aldrich) was added to 180 mL of distilled water, and then dissolved while stirring at 600 rpm under a condition of 85°C and cooled to room temperature to prepare W2 solution. 1.08 g of poly(D,L-lactide-co-glycolid (PLGA, MW 192,000-240,000 Da, Evonik) was added to 15 mL of methylene chloride (dichloromethane, DCM, Sigma-Aldrich) and then dissolved to prepare an oil solution. The prepared W1 solution was added to the oil solution and then sonicated in a water bath-type ultrasonic device for 40 seconds to prepare a first emulsion (water-in-oil). While stirring the prepared W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were rapidly cooled using liquid nitrogen and then freeze-dried to obtain a powder of microspheres.

### 2-2. Preparation of ICG-HSA-loaded PLGA microspheres

To prepare indocyanine green/human serum albumin complex-loaded PLGA microspheres (ICG-HSA-PLGA (without P-188)), 0.75 mg of indocyanine green (Dianogreen Injection, Jeil Pharmaceutical Co., Ltd.) was dissolved in a mixed solution of 0.322 mL of human serum albumin (HSA, SK Plasma) (20% concentration, 64.335 mg) and 1.178 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA was added to 15 mL of methylene chloride and then dissolved to prepare an oil solution. The prepared W1 solution was added to the oil solution and then sonicated in a water bath-type ultrasonic device for 40 seconds to prepare a first emulsion (water-in-oil). While stirring the prepared W2 solution at 1000 rpm using a mechanical stirrer, the first W/O emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were rapidly cooled using liquid nitrogen and then freeze-dried to obtain a powder of microspheres.

### 2-3. Preparation of ICG-HSA-loaded PLGA microspheres comprising surfactant (poloxamer)

To examine the effect when a polymer such as PLGA is additionally mixed with a surfactant in an organic solvent, microspheres were prepared in which a surfactant was added to PLGA. To prepare ICG-HSA-loaded PLGA microspheres (ICG-HSA-PLGA (P-188 0.1%)) containing 0.1% (oil phase) poloxamer 188 (P-188, BASF) as a surfactant, 0.75 mg of indocyanine green (Dianogreen Injection, Jeil Pharmaceutical Co., Ltd.) was dissolved in a mixed solution of 0.322 mL of human serum albumin (HSA, SK Plasma) (20% concentration, 64.335 mg) and 1.178 mL of distilled water to prepare W1 solution. 3.6 g of polyvinyl alcohol was added to 180 mL of distilled water, and then dissolved while stirring at 600 rpm under a condition of 85°C and cooled to room temperature to prepare W2 solution. 1.08 g of PLGA and 16.5 mg of poloxamer 188 (P-188, BASF) were added to 15 mL of methylene chloride and then dissolved to prepare an oil solution. The prepared W1 solution was added to the oil solution and then sonicated in a water bath-type ultrasonic device for 40 seconds to prepare a first emulsion (water-in-oil). While stirring the prepared W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were rapidly cooled using liquid nitrogen and then freeze-dried to obtain a powder of microspheres.

### 2-4. Analysis of indocyanine green content and fluorescence intensity in microspheres

To evaluate the content and loading rate of indocyanine green in the prepared microspheres, the concentration of indocyanine green in the microsphere-dissolved solution was quantified using an ultraviolet/visible light absorption spectrometer. 0.5 mg of indocyanine green was dissolved in a mixed solution of 0.214 mL of human serum albumin (20% concentration, 42.89 mg) and 0.786 mL of distilled water to prepare a high concentration indocyanine green/human serum albumin complex solution. The high concentration complex solution was diluted with distilled water to prepare standard solutions with 13 different concentrations between 0.065 and 6.54 µM. After the absorbance was measured with the standard solution and a calibration curve was created, the absorbance of a solution in which 10 mg of PLGA microspheres was dissolved in 1 mL of dimethyl sulfoxide (DMSO, Daejung Chemicals & Metals Co., Ltd.) solvent was analyzed using an ultraviolet/visible light absorption spectrometer, and the concentration was measured by substituting the calibration curve. The content and loading efficiency of indocyanine green in the microspheres were evaluated by substituting the measured concentration of the complex into the following equation: Content of indocyanine green in microspheres = weight of indocyanine green in 10 mg of microspheres/10 mg (weight of microspheres) Loading rate of indocyanine green in microspheres = (weight of indocyanine green loaded on the entire microspheres/weight of indocyanine green used for loading onto microspheres) × 100%

To analyze the particle shape and size of the synthesized microspheres, scanning electron microscope (JSM-7800F Prime, JEOL Ltd, Japan) analysis was performed. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. 0.5 mg of indocyanine green powder was dissolved in a mixed solution of 0.214 mL of human serum albumin solution (20% concentration) and 0.786 mL of distilled water, and then diluted with distilled water to prepare 0.3 mL of indocyanine green/human serum albumin complex (ICG-HSA) at a concentration of 30 µM. Using the information on the content of indocyanine green in PLGA microspheres evaluated by the quantification, a dispersion was prepared by adding indocyanine green to 0.3 mL of distilled water so that the concentration was 30 µM. To compare fluorescence spectra, 200 µL of each of the prepared solution and dispersion was aliquoted and placed in a 96-well plate, and the fluorescence spectrum (λₑₓ = 730 nm) was measured using a multi-function microplate reader (SPARK, Tecan Trading AG, Zurich, Switzerland).

As a result of quantifying indocyanine green in microspheres, it was confirmed that the loading rate of indocyanine green in ICG-PLGA (without P-188) microspheres was 3.99% and 43.6 ng of indocyanine green was loaded in 1 mg of microspheres. In the case of ICG-HSA-PLGA (without P-188) microspheres, it was confirmed that the loading rate was 8.39% and the content of indocyanine green in 1 mg of microspheres was 93.6 ng. In the case of ICG-HSA-PLGA (P-188 0.1%) prepared under conditions containing 0.1% poloxamer 188 based on the first emulsion when synthesizing ICG-HSA-PLGA, it was confirmed that the loading rate of indocyanine green compared to ICG-HSA-PLGA (without P-188) was 5.26% and the content of indocyanine green in 1 mg of microspheres was reduced to 59.7 ng. This is believed to be because poloxamer 188 having both hydrophilic and hydrophobic functional groups is evenly distributed in PLGA, and the hydrophilic indocyanine green/human serum complex is evenly distributed on PLGA and forms a passage for water to pass through, allowing some of the fluorescent dye to be eluted during the synthesis process.

It was confirmed that the particle shape of the three microspheres identified through scanning electron microscopy images was spherical (Figure 3, (a) ICG-PLGA, (b) ICG-HSA-PLGA, (c) ICG-HSA-PLGA (P-188 0.1%)), and it was confirmed that the average particle size was 67.5 ± 24.6 µm, 85.4 ± 28.7 um and 77.5 ± 20.2 µm in the order of ICG-PLGA (without P-188), ICG-HSA-PLGA (without P-188) and ICG-HSA-PLGA (P-188 0.1%) and all were particles of 20 um or larger, which are capable of avoiding the phagocytosis of macrophages.

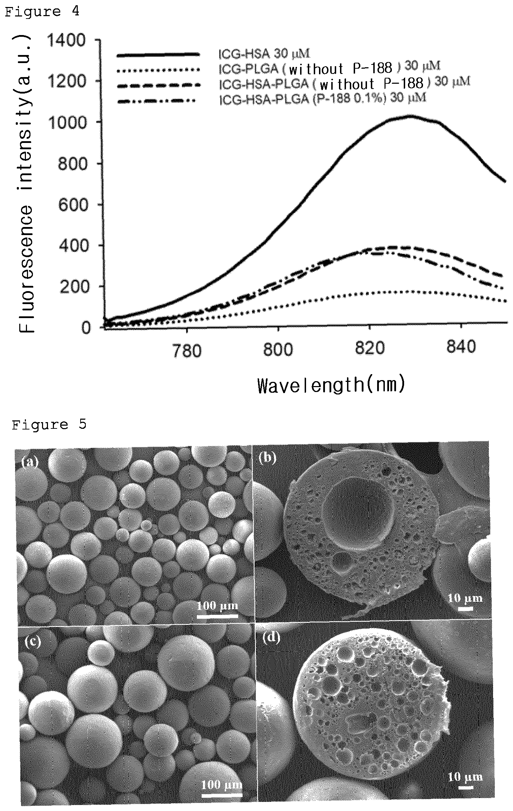

As can be seen from the fluorescence spectrum (Figure 4), in the case of a dispersion of ICG-PLGA (without P-188) microspheres loaded with ICG alone, it could be confirmed that the fluorescence signal was 6.8 times lower than that of ICG-HSA. This is believed to be because indocyanine green does not prevent aggregation of fluorescent dyes when loaded on PLGA, and thus results in a signal reduction effect. Even in the dispersions of ICG-HSA-PLGA (without P-188) and ICG-HSA-PLGA (P-188 0.1%) microspheres, it was confirmed that although the fluorescence signal was decreased to 2.8 times or less compared to ICG-HSA, the fluorescence signal was 2.4 times higher than that of ICG-PLGA (without P-188). From the above, it was confirmed that surgical marker microspheres with a higher fluorescence signal could be obtained when loading ICG-HSA on PLGA rather than loading ICG alone on PLGA. This appears to be because HSA prevents the aggregation of ICG and thus partially prevents the decrease in fluorescence signal. Microspheres prepared to contain 0.1% poloxamer 188 showed a similar fluorescence intensity to PLGA microspheres loaded with ICG-HSA without poloxamer 188, and showed a higher fluorescence signal than ICG-PLGA microspheres.

### Example 3: Preparation and fluorescence intensity analysis of ICG-HSA-PLGA microspheres depending on concentration of poloxamer 188

### 3-1. Preparation and experimental method of microspheres

In this example, microspheres were prepared by increasing the concentration of poloxamer 188, and the effect on the fluorescence intensity of the microspheres was observed. To prepare ICG-HSA-loaded PLGA microspheres (ICG-HSA-PLGA (P-188 0.5%)) containing 0.5% (oil phase) poloxamer 188 (P-188, BASF) based on the first emulsion, 0.75 mg of indocyanine green was dissolved in a mixed solution of 0.322 mL of human serum albumin (20% concentration, 64.335 mg) and 1.178 mL of distilled water to prepare W1 solution. 3.6 g of poly (vinyl alcohol) was added to 180 mL of distilled water, and then dissolved while stirring at 600 rpm under a condition of 85°C and cooled to room temperature to prepare W2 solution. 1.08 g of PLGA and 82.5 mg of poloxamer 188 were added to 15 mL of methylene chloride and then dissolved to prepare an oil solution. The prepared W1 solution was added to the oil solution and then sonicated in a water bath-type ultrasonic device for 40 seconds to prepare a first emulsion (water-in-oil). While stirring the prepared W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were rapidly cooled using liquid nitrogen and then freeze-dried to obtain a powder of microspheres.

To prepare ICG-HSA-loaded PLGA microspheres (ICG-HSA-PLGA (P-188 1.0%)) containing 1.0% (oil phase) poloxamer 188 based on the first emulsion, 0.75 mg of indocyanine green was dissolved in a mixed solution of 0.322 mL of human serum albumin (20% concentration, 64.335 mg) and 1.178 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA and 165 mg of poloxamer 188 were added to 15 mL of methylene chloride and then dissolved to prepare an oil solution. The prepared W1 solution was added to the oil solution and then sonicated in a water bath-type ultrasonic device for 40 seconds to prepare a first emulsion (water-in-oil). While stirring the prepared W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were rapidly cooled using liquid nitrogen and then freeze-dried to obtain a powder of microspheres.

To evaluate the content and loading rate of indocyanine green in the prepared microspheres, the concentration of indocyanine green in the microsphere-dissolved solution was quantified using an ultraviolet/visible light absorption spectrometer. 0.5 mg of indocyanine green was dissolved in a mixed solution of 0.214 mL of human serum albumin (20% concentration, 42.89 mg) and 0.786 mL of distilled water to prepare a high concentration indocyanine green/human serum albumin complex solution. The high concentration complex solution was diluted with distilled water to prepare standard solutions with 13 different concentrations between 0.065 and 6.54 µM. After the absorbance was measured with the standard solution and a calibration curve was created, the absorbance of a solution in which 10 mg of PLGA microspheres was dissolved in 1 mL of dimethyl sulfoxide solvent was analyzed using an ultraviolet/visible light absorption spectrometer, and the concentration was measured by substituting the calibration curve. The content and loading efficiency of indocyanine green in the microspheres were evaluated by substituting the measured concentration of the complex into the following equation:
To analyze the particle shape and size of the synthesized microspheres, scanning electron microscope analysis was performed. For cross-sectional analysis of the prepared microspheres, the powder of the microspheres was pulverized with a razor blade. The powder of the microspheres for measuring the surface and cross section of microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. 0.5 mg of indocyanine green powder was dissolved in a mixed solution of 0.214 mL of human serum albumin solution (20% concentration) and 0.786 mL of distilled water, and then diluted with distilled water to prepare 0.3 mL of indocyanine green/human serum albumin complex (ICG-HSA) at a concentration of 30 µM. Using the information on the content of indocyanine green in PLGA microspheres evaluated by the quantification, a dispersion was prepared by adding indocyanine green to 0.3 mL of distilled water so that the concentration was 30 µM. To compare fluorescence spectra, 200 µL of each of the prepared solution and dispersion was aliquoted and placed in a 96-well plate, and the fluorescence spectrum (λₑₓ = 730 nm) was measured using a multi-function microplate reader. Fluorescence images were analyzed using fluorescence imaging equipment (IVIS Lumina XR, Xenogen Corporation-Caliper, CA, USA) after putting 300 µL of the same solution into a microtube (λₑₓ = 780/20 nm, λₑₘ = 845/40 nm).

To evaluate the fluorescence persistence of surgical marker microspheres in animal models, 100 µL of ICG-HSA solution and dispersions of microspheres at a concentration of 30 µM ICG prepared using physiological saline for injection containing 0.5% sodium carboxymethylcellulose (Sigma-Aldrich) and 0.1% Tween (Tween 80, Sigma-Aldrich) were injected subcutaneously into SKH-1 hairless mice (Orient Bio Co., Ltd.) using a 1 mL syringe (21G needle), and then, fluorescent signals over time were captured using fluorescence imaging equipment (IVIS Lumina XR) (λₑₓ = 780/20 nm, λₑₘ = 845/40 nm).

### 3-2. Comparative analysis of loading rate and fluorescence intensity of indocyanine green

When preparing ICG-HSA-PLGA microspheres, it could be confirmed that the loading rate of ICG in ICG-HSA-PLGA (P-188 0.5%) microspheres prepared by increasing the concentration of poloxamer 188 to 0.5% based on the first emulsion was 13.8% and the content of indocyanine green in 1 mg of microspheres was 124.2 ng and thus, the loading rate and content of indocyanine green increased compared to ICG-HSA-PLGA (P-188 0.1%). When preparing ICG-HSA-PLGA microspheres, it was confirmed that the loading rate of ICG in ICG-HSA-PLGA (P-188 1.0%) microspheres prepared by increasing the concentration of poloxamer 188 to 1.0% based on the first emulsion was 8.71% and the content of indocyanine green in 1 mg of microspheres was 72.7 ng and thus, the loading rate and content of indocyanine green increased compared to ICG-HSA-PLGA (P-188 0.1%) .

As a result of scanning electron microscopy of ICG-HSA-PLGA (P-188 0.5%) and ICG-HSA-PLGA (P-188 1.0%) microspheres, it could be confirmed that the two microspheres were spherical with average particle sizes of 59.8 ± 19.6 µm and 68.0 ± 27.6 µm, respectively (Figure 5, (a, b: ICG-HSA-PLGA (P-188 0.5%), c, d: ICG-HSA-PLGA (P-188 1.0%))), and as a result of evaluating the cross-section through scanning electron microscopy, both of the microspheres had several small spherical spaces inside the PLGA microspheres. In the case of PLGA microbubbles, there is a disadvantage that if the surface thickness is thin and a hole is created due to decomposition of the surface, the dye loaded inside the microbubbles may be quickly eluted, whereas in the case where several spherical spaces loaded with dye are isolated from each other, as in the case of the microspheres prepared in the present invention, there is an advantage that even if a hole is created due to partial decomposition of the surface of the microspheres, elution of the loaded fluorescent dye may be prevented, and the fluorescent dye may be maintained in the microspheres at a high concentration for a long period of time at the marked sited.

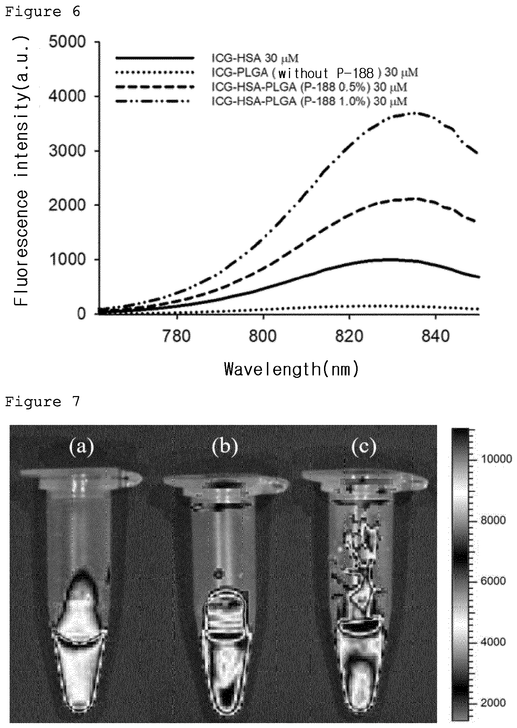

In the fluorescence spectra of ICG-HSA-PLGA (P-188 0.5%) and ICG-HSA-PLGA (P-188 1.0%) dispersions in Figure 6, it could be confirmed that fluorescence signals were 2.1 and 3.6 times higher than ICG-HSA, respectively, and 14 and 25 times higher than ICG-PLGA (without P-188), respectively. From these results, it can be seen that when microspheres containing 0.5% or more of poloxamer 188 based on the first emulsion are prepared, a very high fluorescence signal may be obtained.

Even when the dispersions of microspheres were photographed with a fluorescence imaging device (Figure 7, a: ICG-HSA, b: ICG-HSA-PLGA (P-188 0.5%), c: ICG-HSA-PLGA (P-188 1.0%)), it was confirmed that ICG-HSA-PLGA (P-188 0.5%) and ICG-HSA-PLGA (P-188 1.0%) microspheres generated strong fluorescence signals.

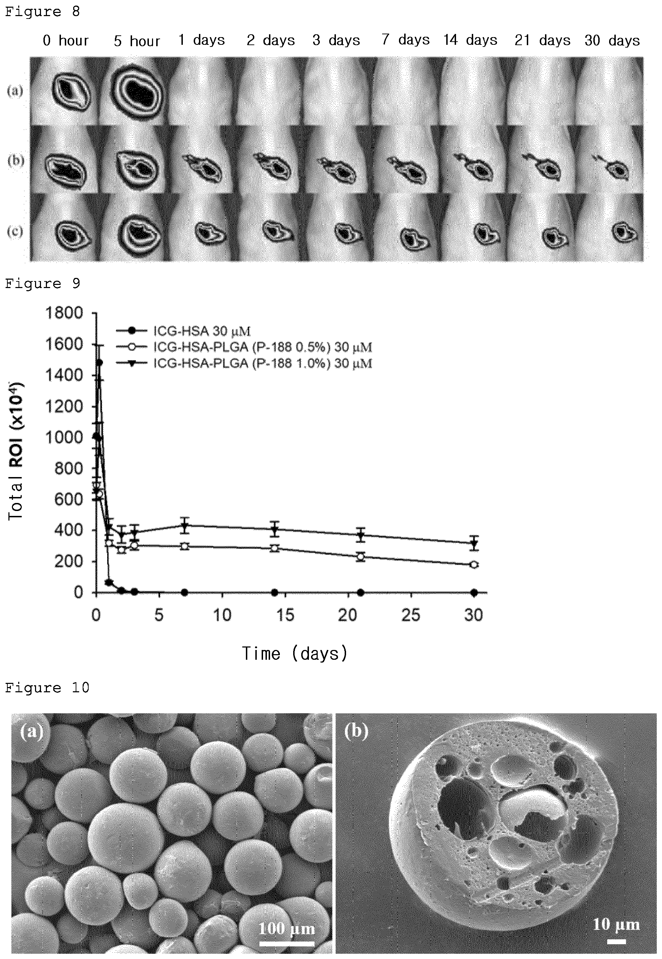

Figure 8 shows fluorescence images over time after subcutaneously injecting ICG-HSA solution and dispersions of ICG-HSA-PLGA (P-188 0.5%) and ICG-HSA-PLGA (P-188 1.0%) microspheres into SKH-1 hairless mice (Δₑₓ = 780/20 nm, λₑₘ = 845/40 nm). When ICG-HSA was injected, it was confirmed that the fluorescence blurring was severe at the administered site, and the fluorescence signal was barely detected 1 day after administration ((a) in Figure 8). On the other hand, in the case of dispersions of ICG-HSA-PLGA (P-188 0.5%) ((b) in Figure 8) and ICG-HSA-PLGA (P-188 1.0%) ((c) in Figure 8) microspheres, it could be seen that the fluorescence signals occurring in the marked sites were slightly reduced until the 1st day, but strong fluorescence signals were maintained without blurring of the fluorescence signals until the 30th day, and the marked sites could be clearly identified from fluorescence imaging.

ROI values for fluorescence signals at marked sites analyzed from the fluorescence images are shown in Figure 9. In the case of ICG-HSA, it was confirmed that the fluorescence signal decreased rapidly after injection, the fluorescence decreased to a level of 6.4% after 1 day and the fluorescence almost disappeared after 2 days.

In the case of dispersions of ICG-HSA-PLGA (P-188 0.5%) and ICG-HSA-PLGA (P-188 1.0%) microspheres, it was confirmed that the highest fluorescence intensity was observed at 5 hours, 46% and 65% of fluorescence remained, respectively, after 1 day, and 26% and 48% of fluorescence still remained after 30 days. From these results, in the case of ICG-HSA-PLGA (P-188 0.5%) and ICG-HSA-PLGA (P-188 1.0%) microspheres, it was confirmed that a strong fluorescence signal could be maintained for 30 days or more without blurring of the fluorescent dye in the marked site.

### Example 4: Preparation and analysis of microspheres using polymers other than PLGA

### 4-1: Preparation and analysis of microspheres using PLGA and PDLLA-PEG block copolymer

To prepare microspheres, poly(D,L-lactide)-block-poly(ethylene glycol) (PDLLA-PEG, Sigma-Aldrich) was used, and the molecular weights of the PDLLA and PEG were 20,000 and 5,000 Da, respectively. To prepare ICG-HSA-Alginate-PLGA and PDLLA-PEG (P-188 0.1%) microspheres, first, 0.75 mg of indocyanine green (ICG) was dissolved in 0.322 mL of human serum albumin (HSA) (20% concentration, 64.335 mg) and 0.278 mL of distilled water, and mixed with a solution of 6 mg of sodium alginate (ALG, Kimica) dissolved in 0.6 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA, 0.12 mg of PDLLA-PEG and 165 mg of poloxamer 188 (P-188) were added to 15 mL of methylene chloride and then dissolved to prepare an oil solution. The prepared W1 solution was added to the oil solution and then sonicated in a water bath-type ultrasonic device for 40 seconds. 0.3 mL of calcium chloride (CaCl₂, Sigma-Aldrich) at a concentration of 4.304 mg/mL was further added and sonicated for 40 seconds to prepare a first emulsion (water-in-oil) in which alginic acid dissolved in W1 was gelled by ionic cross-linking. While stirring the W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were freeze-dried to finally obtain a powder of ICG-HSA-Alginate-PLGA, PDLLA-PEG (P-188 0.1%) microspheres.

To evaluate the content and loading rate of indocyanine green in the prepared microspheres, the microspheres were dissolved and the concentration of indocyanine green in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. 0.5 mg of indocyanine green powder was dissolved in a mixed solution of 0.214 mL of human serum albumin solution (20% concentration) and 0.786 mL of distilled water, and then diluted with distilled water to prepare 0.3 mL of indocyanine green/human serum albumin complex (ICG-HSA) at a concentration of 30 µM. Using the information on the content of indocyanine green in PLGA, PDLLA-PEG microspheres evaluated by the quantification, a dispersion of microspheres was prepared by adding indocyanine green to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader. Fluorescence images were analyzed using fluorescence imaging equipment (IVIS Lumina XR) after putting 300 µL of the same solution into a microtube (λₑₓ = 780/20 nm, λₑₘ = 845/40 nm).

To evaluate changes in fluorescence intensity of injected microspheres in animal models, dispersions of microparticles were prepared using physiological saline for injection containing 0.5% sodium carboxymethylcellulose (Sigma-Aldrich) and 0.1% Tween (Tween 80, Sigma-Aldrich), and 100 µL of these dispersions were injected subcutaneously into SKH-1 hairless mice. Fluorescent signals over time were captured using fluorescence imaging equipment (IVIS Lumina XR) (λₑₓ = 780/20 nm, λₑₘ = 845/40 nm) .

It was confirmed that the loading rate of indocyanine green in ICG-HSA-Alginate-PLGA, PDLLA-PEG (P-188 0.1%) microspheres was 15.8% and the content of indocyanine green per 1 mg of microspheres was 234.6 ng, and thus, the content of indocyanine green increased compared to existing conditions.

It was confirmed that ICG-HSA-Alginate-PLGA, PDLLA-PEG (P-188 0.1%) microspheres were spherical particles with an average particle size of 74.3 ± 18.2 um, and had an appropriate size to avoid the phagocytosis of macrophages ((a) in Figure 10) . In addition, in a scanning electron image of the particle cross-section ((b) in Figure 10), it was confirmed that since the inside of the particle contained several isolated small spherical spaces, just like ICG-HSA-PLGA (P-188 0.5%) or ICG-HSA-PLGA (P-188 1.0%), elution of the loaded fluorescent dye could be prevented, so that it was a very suitable form for long-term fluorescent dye marking.

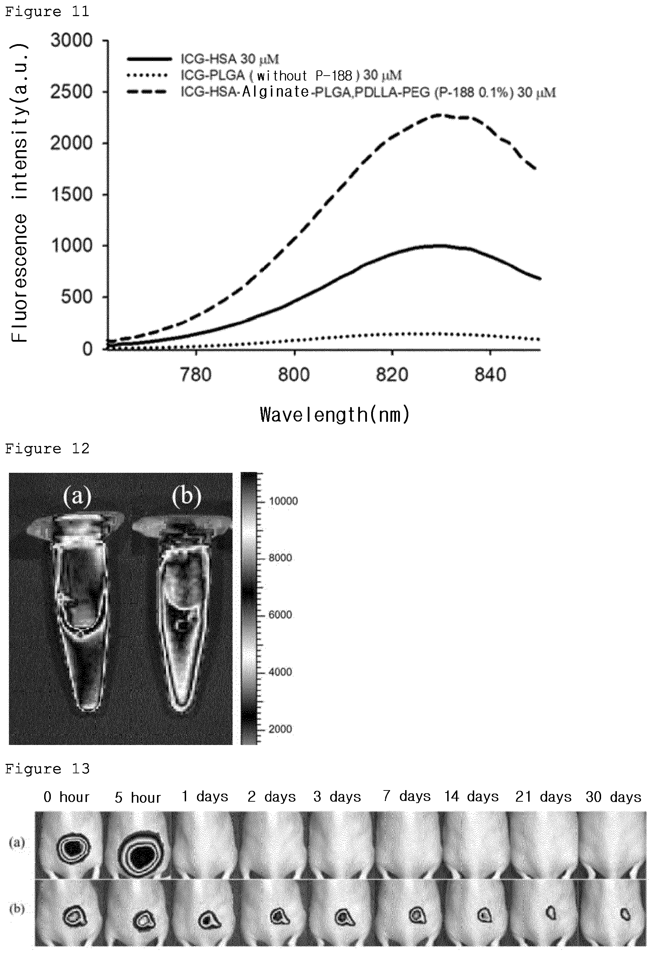

In the fluorescence spectrum of the dispersion of ICG-HSA-Alginate-PLGA, PDLLA-PEG (P-188 0.1%) microspheres in Figure 11, it could be confirmed that the fluorescence signal was 2.3 times higher than ICG-HSA, and 15 times higher than ICG-PLGA (without P-188) .

As a result of comparing the fluorescence images of the dispersion of microspheres (Figure 12), it was confirmed once again that the fluorescence signal of the dispersion of ICG-HSA-Alginate-PLGA, PDLLA-PEG (P-188 0.1%) microspheres ((b) in Figure 12) was higher than that of the ICG-HSA solution ((a) in Figure 12) .

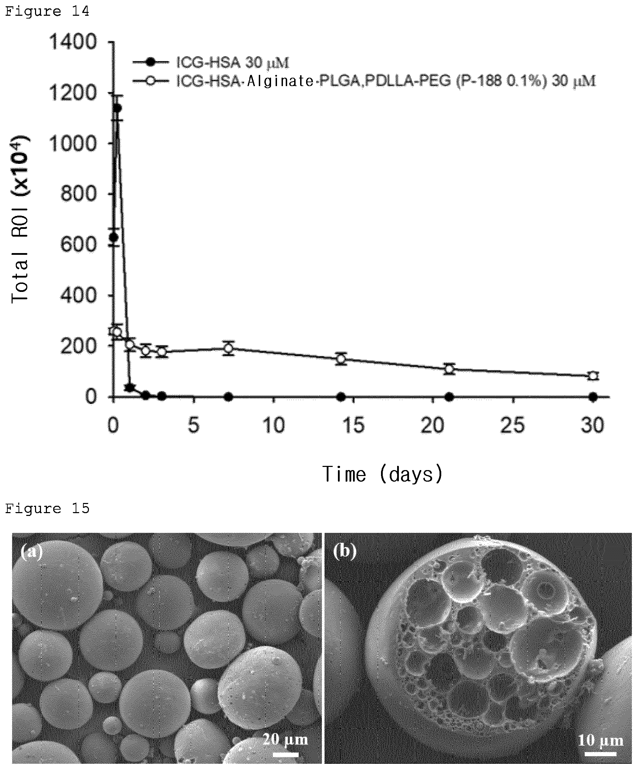

Figure 13 shows fluorescence images taken over time after subcutaneously injecting an ICG-HSA solution and a dispersion of ICG-HSA-Alginate-PLGA, PDLLA-PEG (P-188 0.1%) microspheres into SKH-1 hairless mice (λₑₓ = 780/20 nm, λₑₘ = 845/40 nm). In the case of ICG-HSA, it was confirmed that a fluorescence signal was visible for up to 5 hours, but no fluorescence signal was visible after 1 day ((a) in Figure 13). On the other hand, in the case of the dispersion of ICG-HSA-Alginate-PLGA, PDLLA-PEG (P-188 0.1%) microparticles ((b) in Figure 13), it was confirmed that although the fluorescently marked region gradually decreased after 7 days, the fluorescence signal remained high until 30 days. As a result of graphing the ROI values analyzed from the fluorescence images (Figure 14), unlike ICG-HSA, it was confirmed that when ICG-HSA-Alginate-PLGA, PDLLA-PEG (P-188 0.1%) microspheres were administered, 79% of the fluorescence signal was maintained after 1 day and 32% of the fluorescence signal was maintained after 30 days. This confirmed that the use of ICG-HSA-Alginate-PLGA, PDLLA-PEG (P-188 0.1%) microspheres enabled fluorescent marking of lesion sites for 30 days or longer.

### Example 4-2: Preparation and analysis of microspheres using PMMA polymer

To prepare PMMA microspheres, ICG-HSA-PMMA (P-188 1.0%) microspheres were prepared using poly(methyl methacrylate) (PMMA; MW 120,000 Da, Sigma-Aldrich) polymer. 0.75 mg of indocyanine green (ICG) was dissolved in a mixed solution of 0.322 mL of human serum albumin (HSA) (20% concentration, 64.335 mg) and 1.178 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.08 g of PMMA and 165 mg of poloxamer 188 (P-188) were added to 15 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 30 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To evaluate the content and loading rate of indocyanine green in the prepared microspheres, the microspheres were dissolved and the concentration of indocyanine green in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. 0.5 mg of indocyanine green powder was dissolved in a mixed solution of 0.214 mL of human serum albumin solution (20% concentration) and 0.786 mL of distilled water, and then diluted with distilled water to prepare 0.3 mL of indocyanine green/human serum albumin complex (ICG-HSA) at a concentration of 30 µM. Using the information on the content of indocyanine green in PMMA microspheres evaluated by the quantification, a dispersion of microspheres was prepared by adding indocyanine green to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader. Fluorescence images were analyzed using fluorescence imaging equipment (IVIS Lumina XR) after putting 300 µL of the same solution into a microtube (λₑₓ = 780/20 nm, λₑₘ = 845/40 nm).

To evaluate changes in fluorescence intensity of injected microspheres in animal models, dispersions of microparticles were prepared using physiological saline for injection containing 0.5% sodium carboxymethylcellulose (Sigma-Aldrich) and 0.1% Tween (Tween 80, Sigma-Aldrich), and 100 µL of these dispersions were injected subcutaneously into SKH-1 hairless mice. Fluorescent signals over time were captured using fluorescence imaging equipment (IVIS Lumina XR) (λₑₓ = 780/20 nm, λₑₘ = 845/40 nm) .

The loading rate of indocyanine green in ICG-HSA-PMMA (P-188 1.0%) microspheres was 44.0%, and the content of indocyanine green per 1 mg of microspheres was high at 370.3 ng.

It was confirmed that ICG-HSA-PMMA (P-188 1.0%) microspheres were spherical particles with an average particle size of 49.0 ± 18.7 µm, and had an appropriate size to avoid the phagocytosis of macrophages ((a) in Figure 15). In addition, in a scanning electron image of the particle cross-section ((b) in Figure 15), it was confirmed that since the inside of the particle contained several isolated small spherical spaces, elution of the fluorescent dye loaded in these spaces could be prevented, so that it was a very suitable form for long-term fluorescent dye marking.

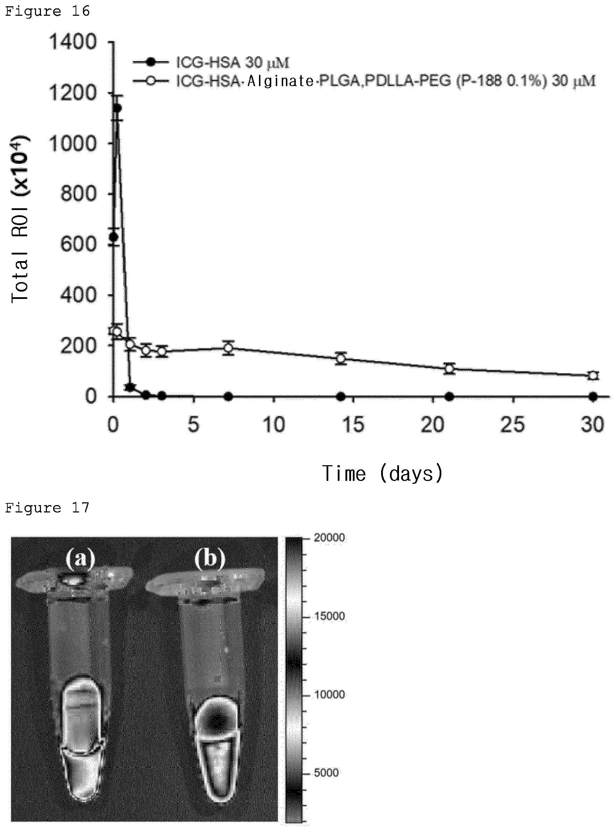

In the fluorescence spectrum of the dispersion of ICG-HSA-PMMA (P-188 1.0%) microspheres in Figure 16, it could be confirmed that the fluorescence signal was at the same level as ICG-HSA and was 7 times higher than ICG-PLGA (without P-188) .

As a result of comparing fluorescence images of the dispersion of ICG-HSA-PMMA (P-188 1.0%) microspheres (Figure 17), it was confirmed that a high fluorescence signal was generated similar to the ICG-HSA solution.

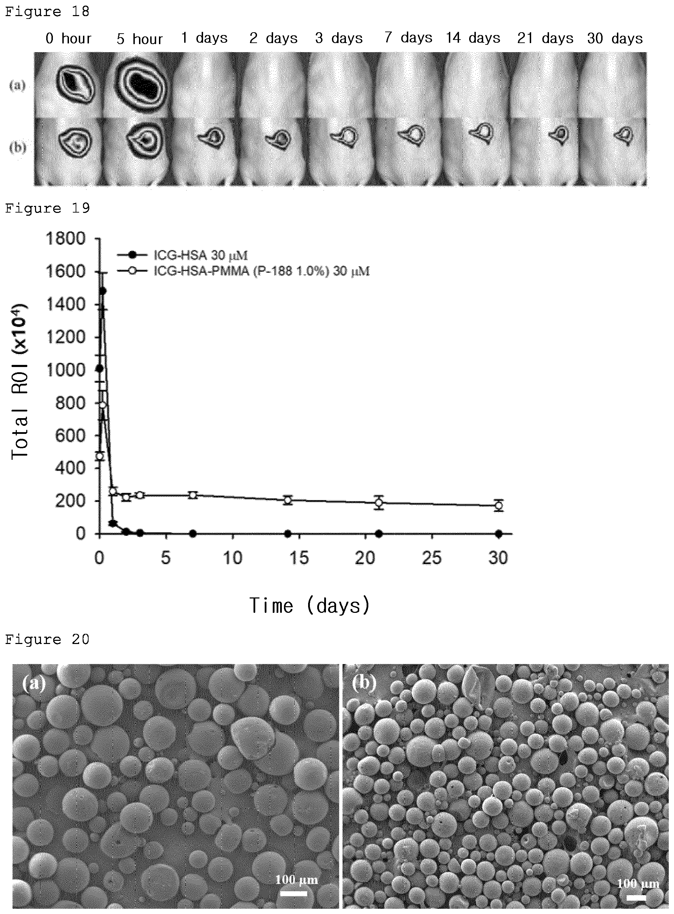

Figure 18 shows the results of fluorescence images taken over time after subcutaneously injecting an ICG-HSA solution and a dispersion of ICG-HSA-PMMA (P-188 1.0%) microspheres into SKH-1 hairless mice (λₑₓ = 780/20 nm, λₑₘ = 845/40 nm). Unlike ICG-HSA ((a) in Figure 18), it was confirmed that when ICG-HSA-PMMA (P-188 1.0%) microspheres ((b) in Figure 18) were administered, a high fluorescence signal was maintained for up to 30 days without any blurring phenomenon of the fluorescent dye.

As a result of graphing the ROI values analyzed from the fluorescence images (Figure 19), unlike ICG-HSA, in the case of the dispersion of ICG-HSA-PMMA (P-188 1.0%) microspheres, it was confirmed that 55% of fluorescence remained after 1 day, and the fluorescence intensity at the level of 36% was maintained even after 30 days. From these results, it was confirmed that ICG-HSA-PMMA (P-188 1.0%) microspheres were useful as a fluorescence marker capable of maintaining a fluorescence signal in the marked lesion for 30 days or more.

From the above examples, it was confirmed that when microspheres are prepared by loading ICG alone, microspheres that generate a very weak fluorescence signal are obtained, and when microspheres are prepared using an ICG-HSA complex, or when ICG-HSA is mixed with an alginate polymer and then gelled through ionic cross-linking, microspheres that generate a high fluorescence signal could be prepared.

### Example 5: Preparation and analysis of ICG-loaded PLGA microspheres using alginate hydrogel

In this example, it was analyzed whether the fluorescence signal of ICG-loaded microspheres could become high even when the W1 solution was prepared by mixing ICG with alginic acid and the alginic acid was ionically cross-linked using calcium. To prepare PLGA microspheres containing ICG in an alginate hydrogel (ICG-Alginate-PLGA (without P-188)), W1 solution was prepared by mixing a solution of 0.75 mg of indocyanine green (ICG) dissolved in 0.6 mL of distilled water and a solution of 6 mg of sodium alginate dissolved in 0.6 mL of distilled water. 3.6 g of poly (vinyl alcohol) was added to 180 mL of distilled water, and then dissolved while stirring at 600 rpm under a condition of 85°C and cooled to room temperature to prepare W2 solution. 1.08 g of PLGA was added to 15 mL of methylene chloride and then dissolved to prepare an oil solution. The prepared W1 solution was added to the oil solution and then sonicated in a water bath type ultrasonic device for 40 seconds, and 0.3 mL of calcium chloride (CaCl₂, Sigma-Aldrich) at a concentration of 4.304 mg/mL was further added to induce ionic cross-linking of alginic acid and sonicated for 40 seconds to prepare a first emulsion (water-in-oil) in which alginic acid was gelled. While stirring the W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, ICG-Alginate-PLGA (without P-188) particles, which are indocyanine green-loaded PLGA polymer microspheres, were obtained by centrifugation at 14,000 rpm for 5 minutes, and the process of decanting the supernatant, adding distilled water, redispersing and centrifuging was repeated three times to wash the microspheres. The particles of the washed microspheres were freeze-dried to obtain a powder of microspheres.

Next, ICG-Alginate-PLGA (P-188 0.1%) microspheres, which are ICG-Alginate-PLGA microspheres further mixed with 0.1% (oil phase) of poloxamer 188 (P-188) based on the first emulsion, were prepared. W1 solution was prepared by mixing a solution of 0.75 mg of indocyanine green (ICG) dissolved in 0.6 mL of distilled water and a solution of 6 mg of sodium alginate dissolved in 0.6 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA and 16.5 mg of poloxamer 188 (P-188) were added to 15 mL of methylene chloride and then dissolved to prepare an oil solution. The prepared W1 solution was added to the oil solution and then sonicated in a water bath type ultrasonic device for 40 seconds, and 0.3 mL of calcium chloride (CaCl₂, Sigma-Aldrich) at a concentration of 4.304 mg/mL was further added to induce the formation of ionic cross-links of alginate polymer and sonicated for 40 seconds to prepare a first emulsion (water-in-oil) in which alginic acid was gelled. While stirring the W2 solution at 1000 rpm using a mechanical stirrer, the prepared first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were freeze-dried to obtain a powder of microspheres.

To evaluate the content and loading rate of indocyanine green in the prepared microspheres, the concentration of indocyanine green in the microsphere-dissolved solution was quantified using an ultraviolet/visible light absorption spectrometer. The absorbance of a solution in which 10 mg of PLGA microspheres was dissolved in 1 mL of dimethyl sulfoxide (DMSO) solvent was analyzed using an ultraviolet/visible light absorption spectrometer, and the concentration of indocyanine green was measured by comparison with the standard calibration curve.

To analyze the particle shape and size of the synthesized microspheres, scanning electron microscope (JSM-7800F Prime) analysis was performed. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. 0.5 mg of indocyanine green powder was dissolved in a mixed solution of 0.214 mL of human serum albumin solution (20% concentration) and 0.786 mL of distilled water, and then diluted with distilled water to prepare 0.3 mL of indocyanine green/human serum albumin complex (ICG-HSA) at a concentration of 30 µM. Using the information on the content of indocyanine green in PLGA microspheres evaluated by the quantification, a dispersion of microspheres was prepared by adding indocyanine green to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader.

The loading rate of indocyanine green in ICG-Algiante-PLGA (without P-188) microspheres is 5.79% and the content of indocyanine green in 1 mg of microspheres is 69.0 ng. In the case of ICG-Algiante-PLGA (P-188 0.1%) microspheres to which 0.1% poloxamer 188 was added based on the first emulsion, the loading rate of indocyanine green was increased to 9.21%, and the content of indocyanine green in 1 mg of microspheres was also increased to 84.3 ng.

In the scanning electron microscopy images in Figure 20, it was confirmed that both ICG-Algiante-PLGA (without P-188) and ICG-Algiante-PLGA (P-188 0.1%) microspheres were spherical particles with average particle sizes of 84.5 ± 33.2 µm and 70.2 ± 21.1 µm, respectively, and through this, it was confirmed that the phagocytosis of macrophages could be effectively avoided.

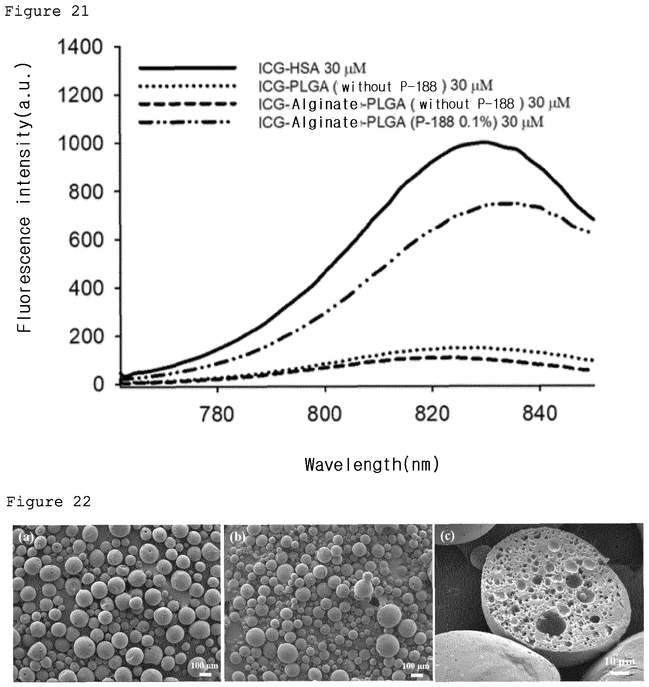

In the fluorescence spectra of the dispersions of ICG-Algiante-PLGA (without P-188) and ICG-Algiante-PLGA (P-188 0.1%) microspheres in Figure 21, it could be confirmed that compared to ICG-PLGA (without P-188) microspheres, ICG-Algiante-PLGA (without P-188) microspheres showed a slight increase in fluorescence, and ICG-Algiante-PLGA (P-188 0.1%) microspheres showed a 5 times higher fluorescence signal. From these results, it could be seen that when poloxamer 188 was added to alginic acid gelation conditions, microspheres showing a high fluorescence signal could be prepared.

### Example 6: Preparation and analysis of PLGA microspheres in which ICG-HSA is mixed in alginate hydrogel

Polymer microspheres were prepared under conditions where the ICG-HSA complex was mixed with alginate polymer and the alginate polymer was ionically cross-linked with calcium, and the fluorescence signal was analyzed.

To prepare ICG-HSA-Alginate-PLGA (P-188 0.1%) microspheres, which are ICG-HSA-loaded PLGA microspheres in which alginic acid is mixed with ICG-HSA and poloxamer 188 (P-188) is added to the oil phase, a W1 solution was prepared by preparing a solution of 75 mg of indocyanine green (ICG) mixed with 0.322 mL of human serum albumin (HSA) (20% concentration, 64.335 mg) and 0.278 mL of distilled water and mixing it with a solution of 6 mg of sodium alginate (ALG, Kimica) dissolved in 0.6 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA and 16.5 mg of poloxamer 188 (P-188) were added to 15 mL of methylene chloride and then dissolved to prepare an oil solution. The prepared W1 solution was added to the oil solution and then sonicated in a water bath type ultrasonic device for 40 seconds, and 0.3 mL of calcium chloride (CaCl₂, Sigma-Aldrich) at a concentration of 4.304 mg/mL was further added and sonicated for 40 seconds to prepare a first emulsion (water-in-oil) in which alginic acid was gelled. While stirring the W2 solution at 1000 rpm using a mechanical stirrer, the first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were freeze-dried to obtain a powder of microspheres.

To prepare ICG-HSA-Alginate-PLGA (P-188 0.5%) microspheres, which are ICG-HSA-loaded PLGA microspheres in which alginic acid is mixed with ICG-HSA and poloxamer 188 (P-188) is added to the oil phase, a W1 solution was prepared by preparing a solution of 75 mg of indocyanine green (ICG) mixed with 0.322 mL of human serum albumin (HSA) (20% concentration, 64.335 mg) and 0.278 mL of distilled water and mixing it with a solution of 6 mg of sodium alginate (ALG, Kimica) dissolved in 0.6 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA and 82.5 mg of poloxamer 188 (P-188) were added to 15 mL of methylene chloride and then dissolved to prepare an oil solution. The prepared W1 solution was added to the oil solution and then sonicated in a water bath type ultrasonic device for 40 seconds, and 0.3 mL of calcium chloride (CaCl₂, Sigma-Aldrich) at a concentration of 4.304 mg/mL was added and further sonicated for 40 seconds to prepare a first emulsion (water-in-oil) in which alginic acid was gelled. While stirring the W2 solution at 1000 rpm using a mechanical stirrer, the first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were freeze-dried to obtain a powder of microspheres.

The content and loading rate of indocyanine green in the prepared microspheres were analyzed.

To analyze the particle shape and size of the synthesized microspheres and to observe the cross-sectional shape, scanning electron microscope analysis was performed. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. To compare fluorescence spectra, 200 µL, of each of the prepared solution and the dispersion of microspheres was aliquoted and placed in a 96-well plate, and the fluorescence spectrum (λₑₓ = 730 nm) was measured using a multi-function microplate reader. Fluorescence images were analyzed using fluorescence imaging equipment (IVIS Lumina XR) after putting 300 µL of the same solution into a microtube (λₑₓ = 780/20 nm, λₑₘ = 845/40 nm) .

To evaluate the fluorescent marking efficacy of fluorescent marker microspheres in animal models, the prepared ICG-HSA solution and microspheres at a concentration of 30 µM ICG were dispersed using physiological saline for injection containing 0.5% sodium carboxymethylcellulose (Sigma-Aldrich) and 0.1% Tween (Tween 80, Sigma-Aldrich), and 100 µL of dispersions of the microspheres were injected subcutaneously into SKH-1 hairless mice using a syringe, and then, fluorescent signals over time were captured using fluorescence imaging equipment (IVIS Lumina XR) (λₑₓ = 780/20 nm, λₑₘ = 845/40 nm) .

The loading rates of indocyanine green in ICG-HSA-Alginate-PLGA (P-188 0.1%) and ICG-HSA-Alginate-PLGA (P-188 0.5%) microspheres were 10.5% and 7.62%, respectively, and the content of indocyanine green in 1 mg of microspheres was analyzed to be 108.7 ng and 61.6 ng.

It was confirmed that ICG-HSA-Alginate-PLGA (P-188 0.1%) and ICG-HSA-Alginate-PLGA (P-188 0.5%) microspheres were spherical particles with average particle sizes of 77.8 ± 26.5 µm and 67.9 ± 24.1 µm (FIG. 22), and had an appropriate size to avoid the phagocytosis of macrophages. In addition, as can be seen in the cross-sectional image of the microspheres in (c) of Figure 22, it could be seen that since the inside of the microsphere is composed of several isolated spherical spaces, the emission of fluorescent dye may be suppressed better than a microbubble-type structure.

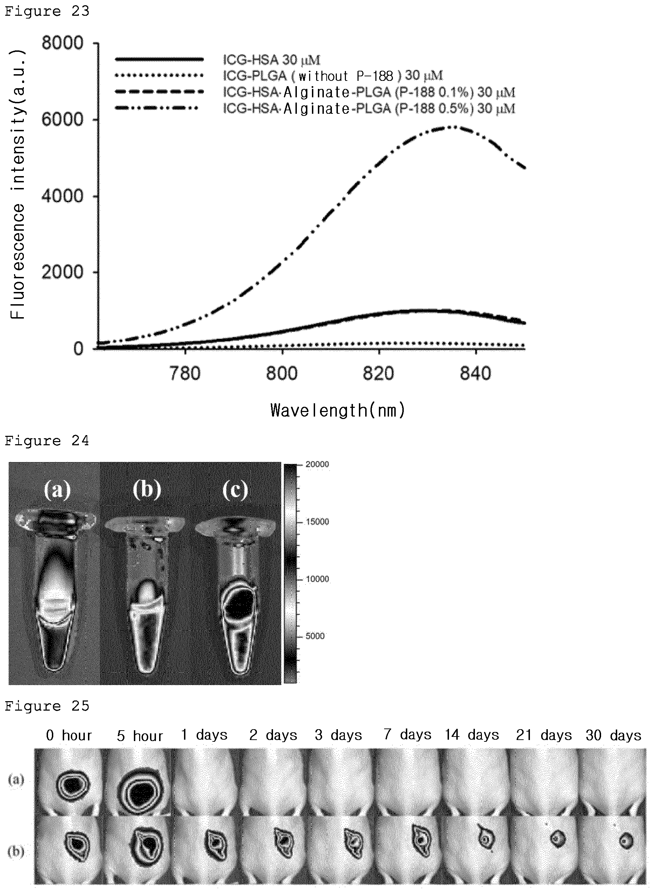

As a result of comparing the fluorescence signal through the fluorescence spectrum in Figure 23, it could be seen that ICG-HSA-Alginate-PLGA (P-188 0.1%) showed a similar fluorescence signal to ICG-HSA and showed a fluorescence signal 6.4 times higher than that of ICG-PLGA (without P-188), and ICG-HSA-Alginate-PLGA (P-188 0.5%) microspheres showed a fluorescence signal 2.5 times higher than that of ICG-HSA and showed a fluorescence signal 17 times higher than that of ICG-PLGA (without P-188) .

Even in Fluorescence images (Figure 24), it could be seen that the prepared dispersions of ICG-HSA-Alginate-PLGA (P-188 0.1%) ((b) in Figure 8C) and ICG-HSA-Alginate-PLGA (P-188 0.5%) ((c) in Figure 8C) microspheres generated a strong fluorescence signal.

Figure 25 shows fluorescence images taken over time after subcutaneously injecting ICG-HSA solution and a dispersion of ICG-HSA-Alginate-PLGA (P-188 0.5%) microspheres into SKH-1 hairless mice (λₑₓ = 780/20 nm, λₑₘ = 845/40 nm) . Unlike the case of ICG-HSA ((a) in Figure 25), in which no fluorescence signal was visible after 1 day, it was confirmed that when a dispersion of ICG-HSA-Alginate-PLGA (P-188 0.5%) microspheres ((b) in Figure 25) was administered, a high fluorescence signal was maintained for up to 30 days.

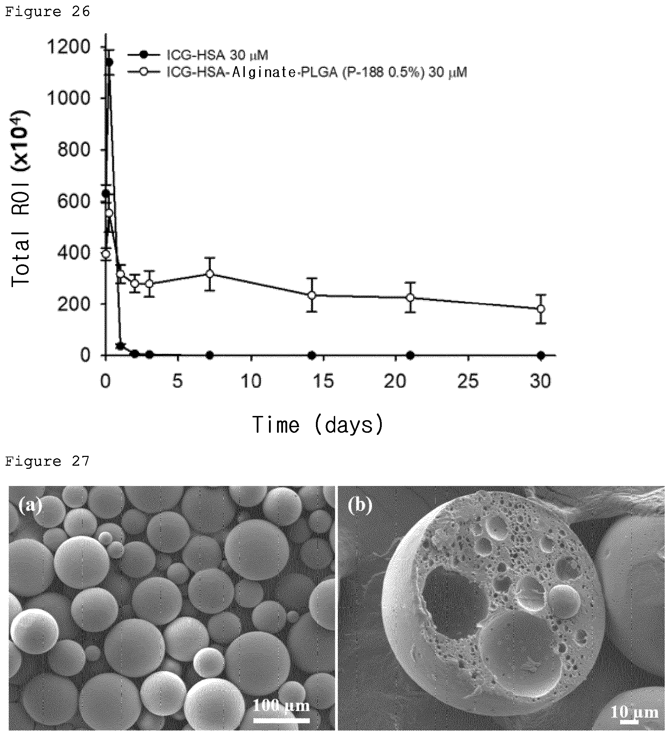

As a result of graphing the ROI values analyzed from the fluorescence images (Figure 26), in the case of the dispersion of ICG-HSA-Alginate-PLGA (P-188 0.5%) microspheres, it was confirmed that 80% of the fluorescence remained after 1 day of administration, and a high fluorescence signal of 46% was visible even after 30 days.

From these results, it was confirmed that ICG-HSA-Alginate-PLGA (P-188) microspheres were very useful as a fluorescent marker.

### Example 7: Preparation and analysis of microspheres using Tween as a surfactant

In this example, in order to confirm whether other types of surfactants could be used instead of P-188 as the surfactant, microspheres were prepared using Tween, and analysis was conducted. W1 solution was prepared by mixing a solution of 0.75 mg of indocyanine green (ICG) mixed with 0.322 mL of human serum albumin (HSA) (20% concentration, 64.335 mg) and 0.278 mL of distilled water and a solution of 6 mg of sodium alginate dissolved in 0.6 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA and 82.5 mg of Tween (Tween 80, Sigma-Aldrich) were added to 15 mL of methylene chloride and then dissolved to prepare an oil solution. The prepared W1 solution was added to the oil solution and then sonicated in a water bath type ultrasonic device for 40 seconds, and 0.3 mL of calcium chloride (CaCl₂, Sigma-Aldrich) at a concentration of 4.304 mg/mL was further added and sonicated for 40 seconds to prepare a first emulsion (water-in-oil) in which alginic acid was gelled. While stirring the W2 solution at 1000 rpm using a mechanical stirrer, the first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The washed microspheres were freeze-dried to obtain a powder of microspheres.

To evaluate the content and loading rate of indocyanine green in the prepared microspheres, the concentration of indocyanine green in the microsphere-dissolved solution was quantified using an ultraviolet/visible light absorption spectrometer. The content and loading efficiency of indocyanine green in the microspheres were evaluated using the measured concentration of the complex.

To analyze the particle shape and size of the synthesized microspheres and the cross section of the microspheres, scanning electron microscope analysis was performed. The powder of the microspheres for analyzing the surface and cross section of microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader. Fluorescence images were analyzed using fluorescence imaging equipment (IVIS Lumina XR) after putting 300 µL of the same solution into a microtube (λₑₓ = 780/20 nm, λₑₘ = 845/40 nm) .

To evaluate the fluorescent marking efficacy of fluorescent marker microspheres in animal models, the prepared ICG-HSA solution and microspheres at a concentration of 30 µM ICG were dispersed using physiological saline for injection containing 0.5% sodium carboxymethylcellulose (Sigma-Aldrich) and 0.1% Tween (Tween 80, Sigma-Aldrich), and 100 µL of dispersions of the microspheres were injected subcutaneously into SKH-1 hairless mice using a syringe, and then, fluorescent signals over time were captured using fluorescence imaging equipment (IVIS Lumina XR) (λₑₓ = 780/20 nm, λₑₘ = 845/40 nm) .

In the case of ICG-HSA-Algiante-PLGA (Tween 0.5%) microspheres in which poloxamer 188, which is a surfactant dissolved in the oil phase, was replaced with Tween in the preparation conditions of ICG-HSA-Algiante-PLGA (P-188 0.5%) microspheres, it was confirmed that the loading rate of indocyanine green in the microspheres was 9.68%, and the content of indocyanine green in 1 mg of microspheres was 81.0 ng.

As can be seen in the scanning electron microscopy images in Figure 27, ICG-HSA-Algiante-PLGA (Tween 0.5%) microspheres had a spherical particle shape ((a) in Figure 27), and the average particle size was 66.1 ± 26.0 µm, which was an appropriate size for evading the phagocytosis of macrophages. In addition, as can be confirmed in the cross-sectional scanning electron microscopy image of the particle ((b) in Figure 27), it was confirmed that since the inside of the microspheres was divided into several isolated small spheres, it had a structure in which the release of the loaded fluorescent dye was suppressed in the same way as the results of the previous examples.

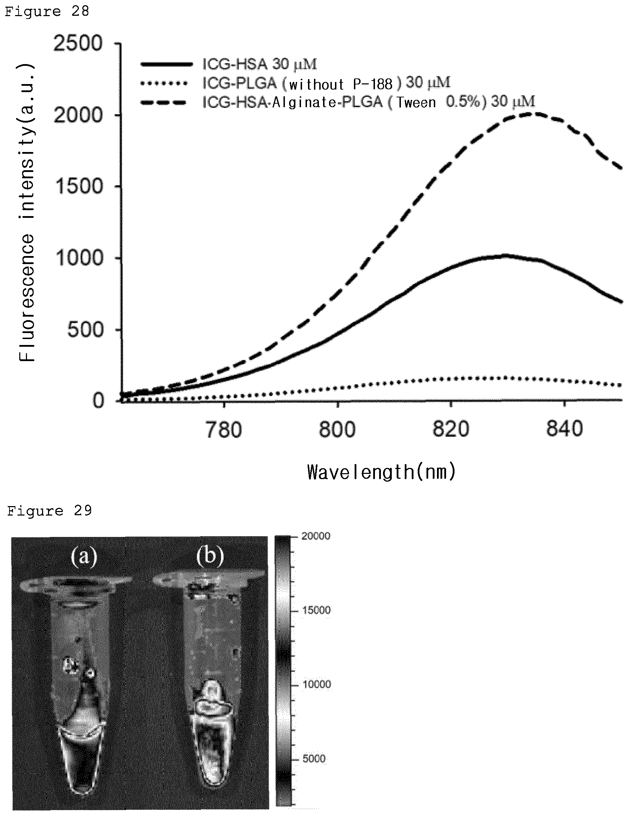

The dispersion of ICG-HSA-Algiante-PLGA (Tween 0.5%) microspheres showed a fluorescence signal that was 2 times higher than that of ICG-HSA, and 13 times higher than that of ICG-PLGA (without P-188) (Figure 28).

From the fluorescence image in Figure 29, it was confirmed that the dispersion of ICG-HSA-Algiante-PLGA (P-188 0.5%) ((b) in Figure 29) microspheres generated a strong fluorescence signal.

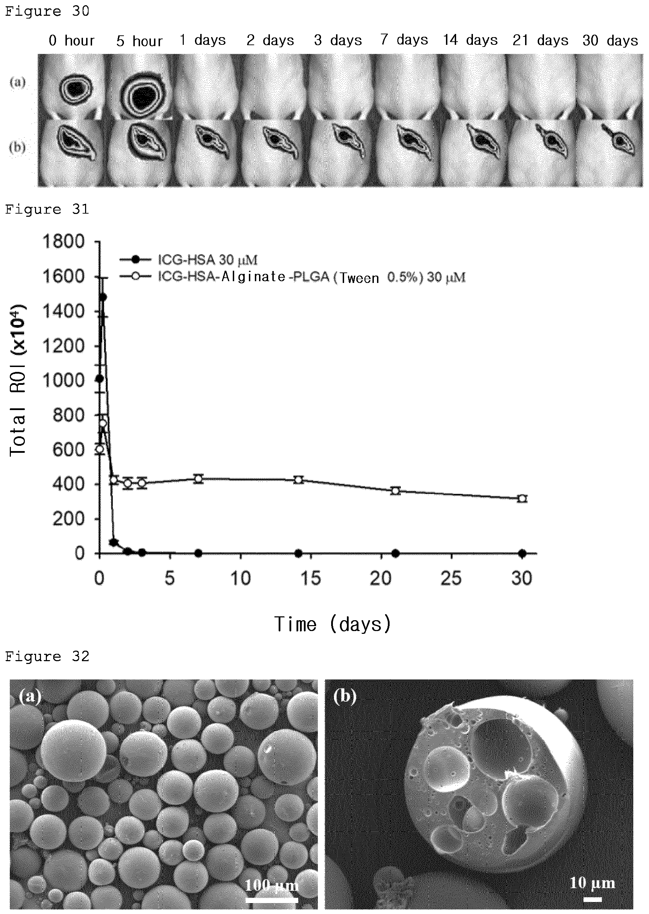

Figure 30 shows fluorescence images taken over time after subcutaneously injecting an ICG-HSA solution ((a) in Figure 30) and a dispersion of ICG-HSA-Alginate-PLGA (Tween 0.5%) microspheres ((b) in Figure 30) into SKH-1 hairless mice (λₑₓ = 780/20 nm, λₑₘ = 845/40 nm). Unlike when ICG-HSA was injected, it was confirmed that in the case of a dispersion of ICG-HSA-Alginate-PLGA (P-188 0.5%) microspheres, the fluorescence signal remained high for 30 days at the administered site without blurring phenomenon of fluorescence to surrounding tissues.

As a result of graphing the ROI values analyzed from the fluorescence images (Figure 31),
in the case of the dispersion of ICG-HSA-Alginate-PLGA (Tween 0.5%) microspheres, it was confirmed that 70% of fluorescence remained after 1 day, and 52% of fluorescence still remained even after 30 days. Based on these results, it can be seen that even if microspheres are prepared by replacing poloxamer 188 as a surfactant with another surfactant such as Tween, microspheres may be prepared that may fluorescently mark a lesion site for a long period of time while generating a high fluorescent signal.

### Example 8: Preparation and analysis of indocyanine green/cyclodextrin complex-loaded microspheres

Indocyanine green was formed into a complex with cyclodextrin (mCD), microspheres loaded therewith were prepared, and the intensity of fluorescence and utility as a fluorescent marker were analyzed. To prepare indocyanine green/cyclodextrin complex-loaded PLGA microspheres (ICG-mCD-Alginate-PLGA (P-188 0.1%)), a solution of 0.375 mg of indocyanine green (ICG) dissolved in 0.3 mL of ethanol and a solution of 1.790 mg of methylcyclodextrin (mCD, Sigma-Aldrich) dissolved in 0.6 mL of distilled water were mixed and stirred for 6 hours. It was mixed with a solution of 3.6 mg of sodium alginate dissolved in 0.648 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.08 g of PLGA and 16.5 mg of poloxamer 188 (P-188) were added to 15 mL of methylene chloride and then dissolved to prepare an oil solution. The prepared W1 solution was added to the oil solution and then sonicated in a water bath type ultrasonic device for 40 seconds, and 0.072 mL of calcium chloride (CaCl₂, Sigma-Aldrich) at a concentration of 10.76 mg/mL was added and further sonicated for 40 seconds to prepare a first emulsion (water-in-oil) in which alginic acid was gelled. While stirring the W2 solution at 1000 rpm using a mechanical stirrer, the first emulsion was added thereto to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of indocyanine green-loaded PLGA polymer microspheres were obtained by centrifugation at 14,000 rpm for 5 minutes, and the process of decanting the supernatant, adding distilled water, redispersing and centrifuging was repeated three times to wash the microspheres. The particles of the washed microspheres were freeze-dried to obtain a powder of microspheres.

The content and loading rate of indocyanine green in the prepared microspheres were analyzed.

To analyze the particle shape and size of the synthesized microspheres and to observe the cross-sectional shape, scanning electron microscope analysis was performed. The particle size was calculated as the average value by randomly measuring the size of 100 particles from the acquired images.

To compare the fluorescence signals of the prepared microspheres and the indocyanine green/human serum albumin complex, the same concentration of indocyanxhine green complex solution and dispersion of microparticles were prepared. 0.5 mg of indocyanine green powder was dissolved in a mixed solution of 0.214 mL of human serum albumin solution (20% concentration) and 0.786 mL of distilled water, and then diluted with distilled water to prepare 0.3 mL of indocyanine green/human serum albumin complex (ICG-HSA) at a concentration of 30 µM. Using the information on the content of indocyanine green in PLGA microspheres evaluated by the quantification, a dispersion of microspheres was prepared by adding indocyanine green to 0.3 mL of distilled water so that the concentration was 30 µM. To compare fluorescence spectra, 200 µL of each of the prepared solution and dispersion was aliquoted and placed in a 96-well plate, and the fluorescence spectrum (λₑₓ = 730 nm) was measured using a multi-function microplate reader. Fluorescence images were analyzed using fluorescence imaging equipment (IVIS Lumina XR) after putting 300 µL of the same solution into a microtube (λₑₓ = 780/20 nm, λₑₘ = 845/40 nm) .

To evaluate the fluorescent marking efficacy of fluorescent marker microspheres in animal models, the prepared ICG-HSA solution and microspheres at a concentration of 30 µM ICG were dispersed using physiological saline for injection containing 0.5% sodium carboxymethylcellulose (Sigma-Aldrich) and 0.1% Tween (Tween 80, Sigma-Aldrich), and 100 µL of dispersions of the microspheres were injected subcutaneously into SKH-1 hairless mice using a syringe, and then, fluorescent signals over time were captured using fluorescence imaging equipment (IVIS Lumina XR) (λₑₓ = 780/20 nm, λₑₘ = 845/40 nm) .

The loading rates of indocyanine green in indocyanine green/cyclodextrin-loaded ICG-mCD-Alginate-PLGA (P-188 0.1%) microspheres were 11.4%, and the content of indocyanine green in 1 mg of microspheres was confirmed to be 76.3 ng.

ICG-mCD-Alginate-PLGA (P-188 0.1%) microspheres were also spherical particles ((a) in Figure 32), with an average size of 65.0 ± 26.0 µm, which was an appropriate size for evading the phagocytosis of macrophages. In addition, as can be seen in the cross-sectional image of the microspheres in (b) of Figure 32, it could be seen that since the inside of the microsphere is composed of several isolated spherical spaces, the emission of fluorescent dye may be suppressed better than a microbubble-type structure.

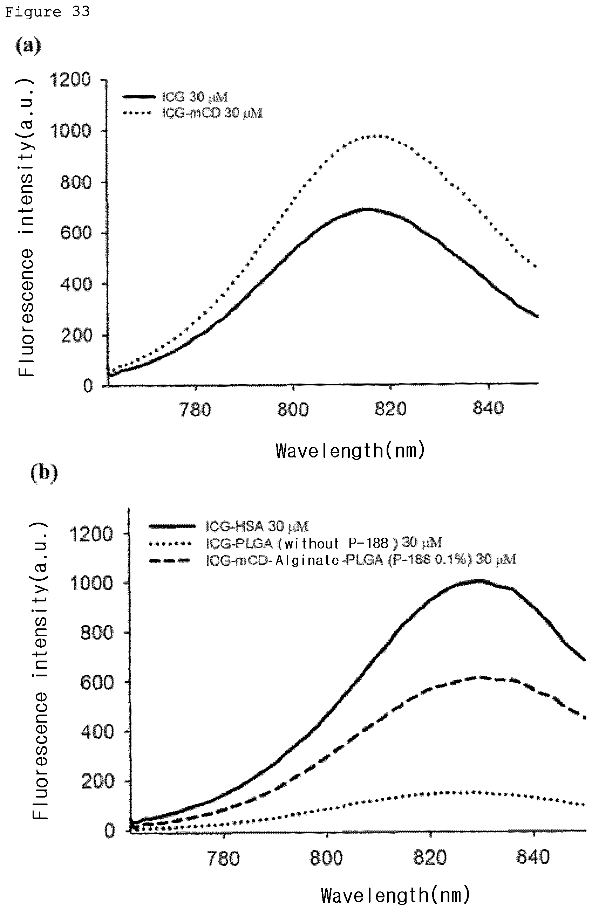

As can be seen in the fluorescence spectrum in (a) of Figure 33, it could be seen that ICG-mCD showed a fluorescence signal 1.5 times higher than that of ICG, which means that the formation of a complex with mCD may prevent the aggregation phenomenon between ICGs in an aqueous solution and also increase the chemical/physical stability of ICG.

It was confirmed that the dispersion of ICG-mCD-Algiante-PLGA (P-188 0.1%) microspheres prepared using the ICG-mCD complex showed a fluorescence signal 4.2 times higher than the dispersion of ICG-PLGA (without P-188) microspheres.

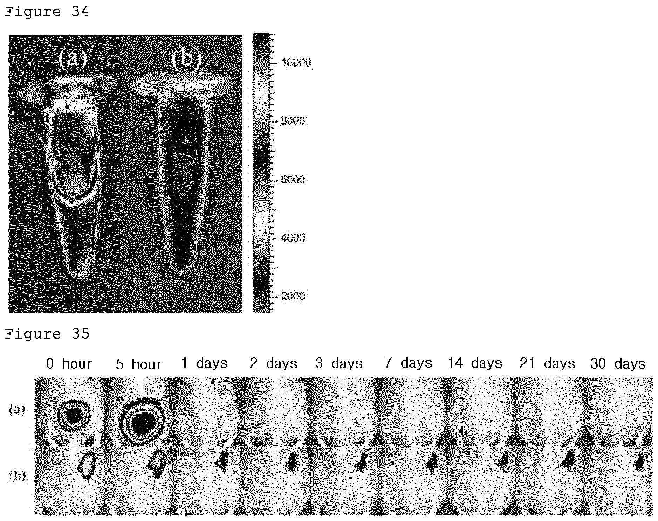

In addition, from the fluorescence images in Figure 34, it was confirmed that the dispersion of ICG-mCD-Algiante-PLGA microspheres (right) generated a high fluorescence signal.

In Figure 35, it was confirmed that the dispersion of ICG-mCD-Algiante-PLGA (P-188 0.1%) microspheres ((b) in Figure 35) maintained the fluorescence signal for up to 30 days without blurring phenomenon of fluorescence at the administered site. ICG-HSA ((a) in Figure 35) showed the same pattern as the previous examples.

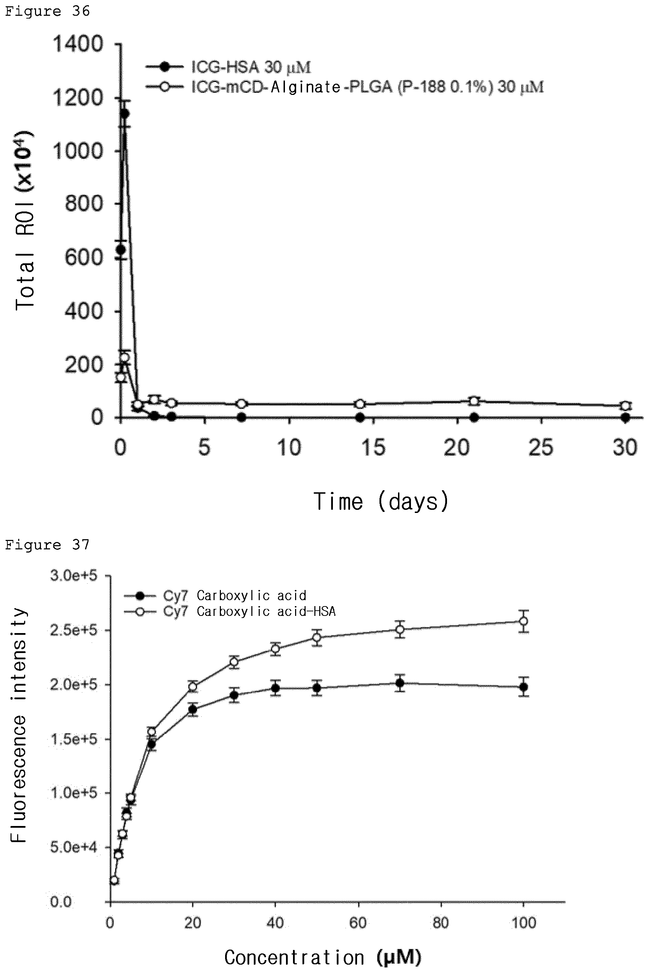

As a result of graphing the ROI values analyzed from the fluorescence images (Figure 36),
in the case of the dispersion of ICG-mCD-Algiante-PLGA (P-188 0.1%) microspheres, it was confirmed that 32% of fluorescence remained after 1 day of injection, and 29% of fluorescence still remained high even after 30 days. From these results, it was confirmed that indocyanine green/cyclodextrin complex-loaded (ICG-mCD-Algiante-PLGA (P-188 0.1%)) microspheres could also maintain a fluorescence signal for 30 days or more and were useful as a fluorescence marker.

### Example 9: Preparation and analysis of Cy7-human serum albumin complex-loaded microspheres

In addition to indocyanine green, a proof experiment was conducted showing that other near-infrared fluorescent dye-loaded microspheres may be also prepared and generate bright fluorescence. For this purpose, sulfo-cyanine 7 carboxylic acid (Cy7), sulfo-cyanine 7.5 carboxylic acid (Cy7.5), Flamma 774 carboxylic acid (Flamma774), Alexa fluor 750 carboxylic acid (Alexa 750) and IRDye 800CW carboxylate (IRDye 800CW) were used as fluorescent dyes loaded into the microspheres, and microspheres were prepared using poly(methyl methacrylate) and polycarbonate (PC) polymers, and their efficacy was evaluated.

### 9-1: Comparison of fluorescence signals depending on the concentration of Cy7 solution and Cy7-HSA complex

0.48 mg of sulfo-cyanine7 carboxylic acid (Cy7, Lumiprobe) as a near-infrared fluorescent dye was dissolved in 1 mL of distilled water, and then diluted with distilled water to prepare Cy7 solutions at concentrations of 100, 70, 50, 40, 30, 20, 10, 5, 4, 3, 2 and 1 µM. Cy7-human serum albumin (HSA, SK Plasma) complex was prepared for comparison of fluorescence signal with Cy7 solution. 0.48 mg of Cy7 powder was dissolved in a mixed solution of 0.214 mL of human serum albumin solution (20% concentration) and 0.786 mL of distilled water, and then diluted with distilled water to prepare Cy7-human serum albumin complexes (Cy7-HSA) at concentrations of 100, 70, 50, 40, 30, 20, 10, 5, 4, 3, 2 and 1 µM. To compare fluorescence spectra, 200 µL of each prepared solution was aliquoted and placed in a 96-well plate, and the fluorescence intensity (λₑₓ = 730 nm, λₑₘ = 780 nm (Cy7), λₑₘ = 786 nm (Cy7-HSA)) was measured using a multi-function microplate reader (SPARK, Tecan Trading AG, Zurich, Switzerland). Cy7 fluorescent dye showed an emission peak at 780 nm, but after forming a complex with HAS, it showed an emission peak at 786 nm.

As shown in Figure 37, which compares the fluorescence intensity of the Cy7 solution and the Cy7-HSA complex solution at a concentration of 1-100 µM, it could be confirmed that the Cy7-HSA solution generated a higher fluorescence signal than the Cy7 solution at a concentration of 10 µM or more, and it was confirmed that the Cy7.5-HSA solution generated a fluorescence signal that was 1.2 times higher than the Cy7.5 solution at a concentration of 30 µM and 1.3 times higher at a concentration of 100 µM, which is the maximum concentration measured.

### 9-2: Preparation and analysis of microspheres loaded with Cy7 and Cy7-HSA complex

To prepare PMMA microspheres loaded with Cy7 as a near-infrared fluorescent dye (Cy7-PMMA (without P-188)), 0.81 mg of Cy7 was dissolved in 1.691 mL of distilled water to prepare W1 solution. 3.6 g of poly (vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To prepare PMMA microspheres loaded with Cy7-HSA complex as a near-infrared fluorescent dye (Cy7-HSA-PMMA (without P-188)), 0.81 mg of Cy7 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To prepare PMMA microspheres (Cy7-HSA-PMMA (P-188 1.0%)) containing poloxamer 188 at a concentration of 1.0% in the first emulsion and loaded with Cy7-HSA complex as a near-infrared fluorescent dye, 0.81 mg of Cy7 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA and 186 mg of poloxamer 188 (P-188) were added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To evaluate the content and loading rate of Cy7 in the prepared microspheres, the microspheres were dissolved and the concentration of Cy7 in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the Cy7-human serum albumin complex, the same concentration of Cy7 complex solution and dispersion of microparticles were prepared. 0.48 mg of Cy7 powder was dissolved in a mixed solution of 0.214 mL of human serum albumin solution (20% concentration) and 0.786 mL of distilled water, and then diluted with distilled water to prepare 0.3 mL of Cy7-human serum albumin complex (Cy7-HSA) at a concentration of 30 µM. Using the information on the content of Cy7 in PMMA microspheres evaluated by the quantification, a dispersion of microspheres was prepared by adding Cy7 to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader.

When preparing PMMA microspheres, it was confirmed that the loading rate of Cy7 in Cy7-PMMA (without P-188) microspheres prepared by loading Cy7 alone was 6.98%, and the content of Cy7 in 1 mg of microspheres was 46.5 ng. It was confirmed that the loading rates of Cy7 in Cy7-HSA-PMMA (without P-188) prepared by loading Cy7-HSA and Cy7-HSA-PMMA (1.0% P-188) microspheres prepared by adding 1.0% poloxamer 188 based on the first emulsion were 25.8% and 19.1%, and the contents of Cy7 in 1 mg of microspheres were 162.0 ng and 104.8 ng, and thus, they were loaded with a higher content of Cy7 than Cy7-PMMA (without P-188) microspheres loaded with Cy7 alone.

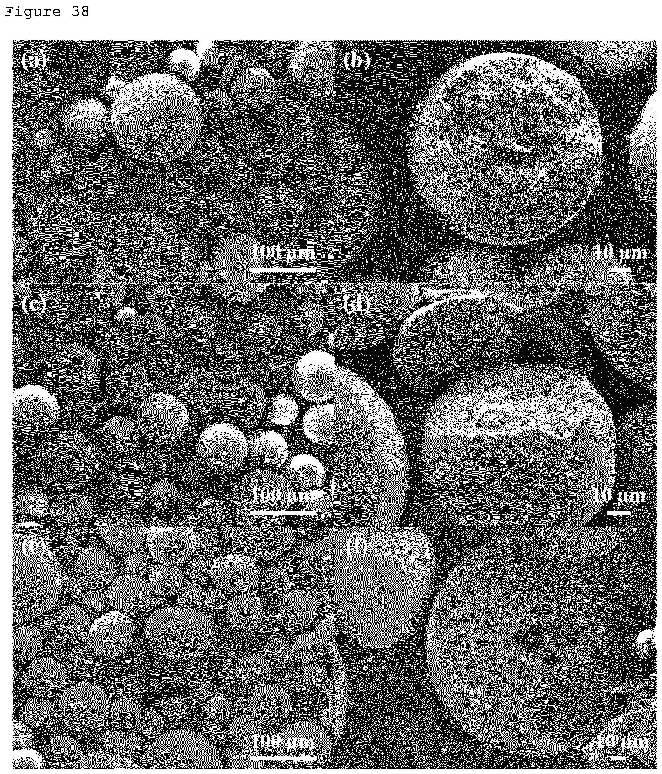

As a result of analyzing scanning electron microscopy images of Cy7-PMMA (without P-188), Cy7-HSA-PMMA (without P-188) and Cy7-HSA-PMMA (1.0% P-188) microspheres, it was confirmed that each of the microspheres was a spherical particle with an average particle size of 56.7 ± 21.3 µm, 53.7 ± 16.1 µm and 59.0 ± 20.0 µm, and had an appropriate size to avoid the phagocytosis of macrophages ((a), (c) and (d) in Figure 38). In addition, as a result of observing the scanning electron microscopy cross-section of each of the microspheres ((b), (d) and (f) in Figure 38), it was confirmed that since the inside of the PMMA microspheres contained several isolated small spherical spaces, elution of the loaded fluorescent dye could be prevented, so that they were a very suitable form for long-term fluorescent dye marking.

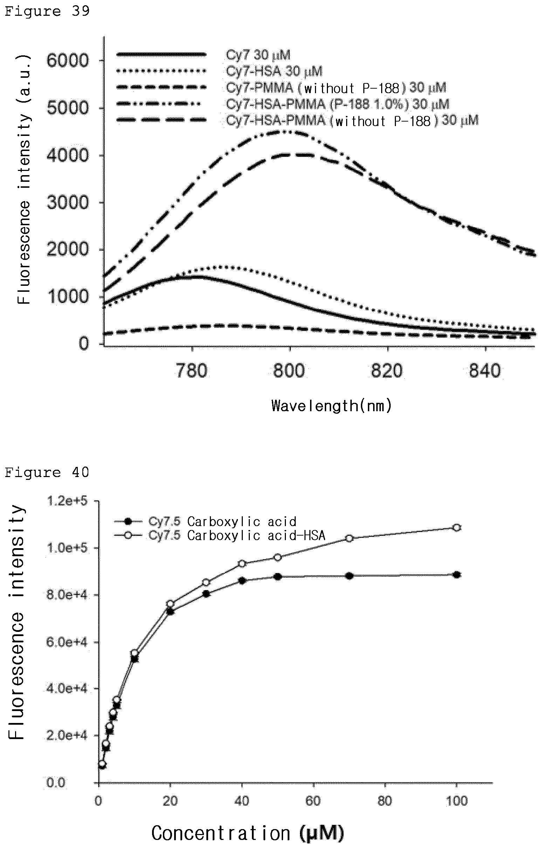

In the fluorescence spectrum of the dispersion of Cy7-PMMA (without P-188) microspheres in Figure 39, a fluorescence signal 4.2 times lower than that of Cy7-HSA could be confirmed, and in the fluorescence spectra of the dispersions of Cy7-HSA-PMMA (without P-188) and Cy7-HSA-PMMA (1.0% P-188) microspheres, fluorescence signals that were 2.5 and 2.8 times higher than that of Cy7-HSA, respectively, and 10 and 12 times higher than that of Cy7-PMMA (without P-188) could be confirmed. From these results, it can be seen that a much higher fluorescence signal may be obtained when Cy7 as a near-infrared fluorescent dye is loaded in the form of a Cy7-HSA complex than when loaded alone.

### Example 10: Preparation and analysis of Cy7.5-human serum albumin complex-loaded microspheres

### 10-1: Comparison of fluorescence signals depending on the concentration of Cy7.5 solution and Cy7.5-HSA complex

0.70 mg of sulfo-cyanine7.5 carboxylic acid (Cy7.5, Lumiprobe) as a near-infrared fluorescent dye was dissolved in 1 mL of distilled water, and then diluted with distilled water to prepare Cy7.5 solutions at concentrations of 100, 70, 50, 40, 30, 20, 10, 5, 4, 3, 2 and 1 µM. For Cy7.5-human serum albumin (HSA, SK Plasma) complex for comparison of fluorescence signals with Cy7.5 solution, 0.70 mg of Cy7.5 powder was dissolved in a mixed solution of 0.214 mL of human serum albumin solution (20% concentration) and 0.786 mL of distilled water, and then diluted with distilled water to prepare Cy7.5-human serum albumin complexes (Cy7.5-HSA) at concentrations of 100, 70, 50, 40, 30, 20, 10, 5, 4, 3, 2 and 1 µM. To compare fluorescence spectra, 200 µL of each prepared solution was aliquoted and placed in a 96-well plate, and the fluorescence spectrum (λₑₓ = 730 nm, λₑₘ = 810 nm (Cy7.5), λₑₘ = 824 nm (Cy7.5-HSA)) was measured using a multi-function microplate reader (SPARK, Tecan Trading AG, Zurich, Switzerland). Cy7.5 fluorescent dye showed an emission peak at 810 nm, but after forming a complex with HAS, it showed an emission peak at 824 nm.

As shown in Figure 40, which compares the fluorescence intensity of the Cy7.5 solution and the Cy7.5-HSA complex solution at a concentration of 1-100 µM, it could be confirmed that the Cy7.5-HSA solution generated a higher fluorescence signal than the Cy7.5 solution at a concentration of 10 µM or more, and it was confirmed that the Cy7.5-HSA solution generated a fluorescence signal that was 1.1 times higher than the Cy7.5 solution at a concentration of 30 µM and 1.2 times higher at a concentration of 100 µM, which is the maximum concentration measured.

### 10-2: Preparation and analysis of microspheres loaded with Cy7.5 and Cy7.5-human serum albumin complex

To prepare PMMA microspheres loaded with Cy7.5 alone as a near-infrared fluorescent dye (Cy7.5-PMMA (without P-188)), 1.18 mg of Cy7.5 was dissolved in 1.691 mL of distilled water to prepare W1 solution. 3.6 g of poly (vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7.5-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

Cy7.5-HSA-PMMA (without P-188), which is PMMA microspheres loaded with Cy7.5-HSA complex as a near-infrared fluorescent dye, was prepared. 1.18 mg of Cy7.5 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7.5-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

Cy7.5-HSA-PMMA (P-188 1.0%), which is PMMA microspheres comprising poloxamer 188 at a concentration of 0.1% in the first emulsion and loaded with Cy7.5-HSA complex as a near-infrared fluorescent dye, was prepared. 1.18 mg of Cy7.5 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA and 186 mg of poloxamer 188 (P-188) were added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7.5-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To evaluate the content and loading rate of Cy7.5 in the prepared microspheres, the microspheres were dissolved and the concentration of Cy7.5 in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the Cy7.5-human serum albumin complex, the same concentration of Cy7.5 complex solution and dispersion of microparticles were prepared. 0.70 mg of Cy7.5 powder was dissolved in a mixed solution of 0.214 mL of human serum albumin solution (20% concentration) and 0.786 mL of distilled water, and then diluted with distilled water to prepare 0.3 mL of Cy7.5-human serum albumin complex (Cy7-HSA) at a concentration of 30 µM. Using the information on the content of Cy7.5 in PMMA microspheres evaluated by the quantification, a dispersion of microspheres was prepared by adding Cy7.5 to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader.

When preparing PMMA microspheres, it was confirmed that the loading rate of Cy7.5 in Cy7.5-PMMA (without P-188) microspheres prepared by loading Cy7.5 was 4.10%, and the content of Cy7.5 in 1 mg of microspheres was 39.8 ng. It was confirmed that the loading rates of Cy7.5 in Cy7.5-HSA-PMMA (without P-188) prepared by loading Cy7.5-HSA and Cy7.5-HSA-PMMA (1.0% P-188) microspheres prepared by adding 1.0% poloxamer 188 based on the first emulsion were 14.0% and 15.6%, and the contents of Cy7.5 in 1 mg of microspheres were 128.7 ng and 124.7 ng, and thus, they were loaded with a higher content of Cy7.5 than Cy7.5-PMMA (without P-188) microspheres loaded with Cy7.5 alone.

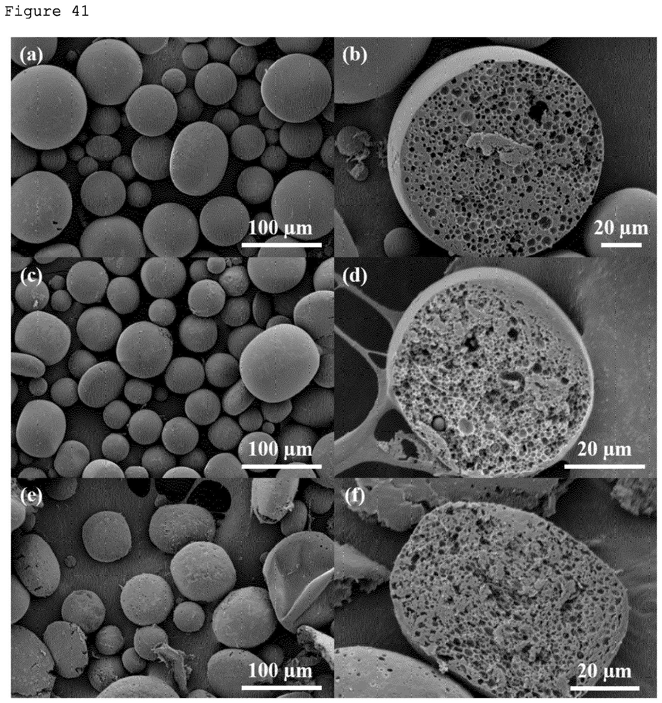

As a result of analyzing scanning electron microscopy images of Cy7.5-PMMA (without P-188), Cy7.5-HSA-PMMA (without P-188) and Cy7.5-HSA-PMMA (1.0% P-188) microspheres, it was confirmed that each of the microspheres was a spherical particle with an average particle size of 57.9 ± 18.4 um, 58.0 ± 16.0 µm and 65.9 ± 24.4 µm, and had an appropriate size to avoid the phagocytosis of macrophages ((a), (c) and (e) in Figure 41). In addition, as a result of evaluating the scanning electron microscopy cross-section of each of the microspheres ((b), (d) and (f) in Figure 41), it was confirmed that since the inside of the PMMA microspheres contained several isolated small spherical spaces, elution of the loaded fluorescent dye could be prevented, so that they were a very suitable form for long-term fluorescent dye marking.

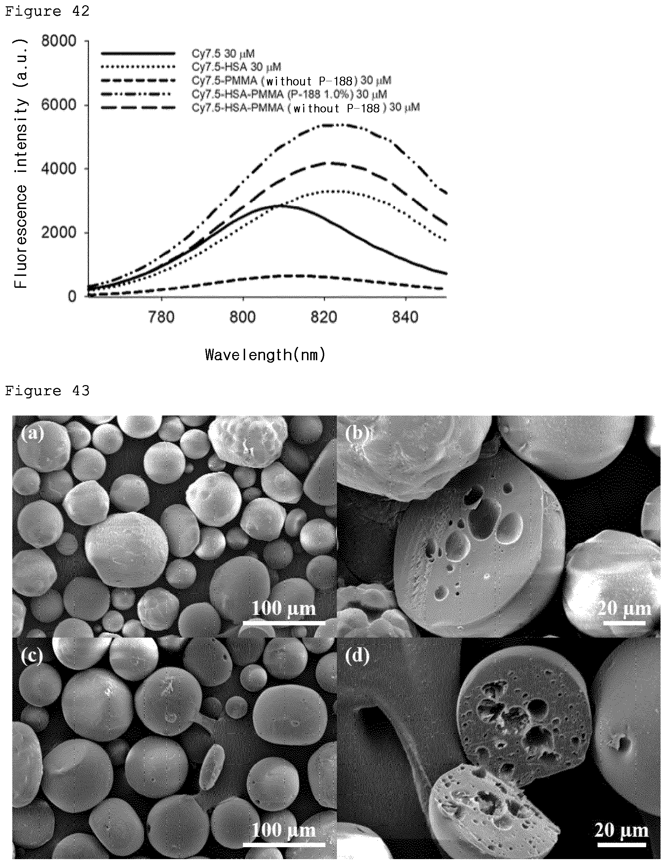

In the fluorescence spectrum of the dispersion of Cy7.5-PMMA (without P-188) microspheres in Figure 42, a fluorescence signal 5.0 times lower than that of Cy7.5-HSA could be confirmed, and in the fluorescence spectra of the dispersions of Cy7.5-HSA-PMMA (without P-188) and Cy7.5-HSA-PMMA (1.0% P-188) microspheres, fluorescence signals that were 1.3 and 1.6 times higher than that of Cy7.5-HSA, respectively, and 6.4 and 8.2 times higher than that of Cy7.5-PMMA (without P-188) could be confirmed. From these results, it can be seen that a much higher fluorescence signal may be obtained when Cy7.5 as a near-infrared fluorescent dye is also loaded in the form of a Cy7.5-HSA complex than when loaded alone.

### 10-3: Preparation and analysis of polycarbonate microspheres loaded with Cy7.5 and Cy7.5-HSA

Cy7.5-PC (without P-188), which is polycarbonate (PC) microspheres loaded with Cy7.5 as a near-infrared fluorescent dye, was prepared. W1 solution was prepared by dissolving 1.18 mg of Cy7.5 in 1.691 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.217 g of PC was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7.5-loaded PC polymer microspheres were obtained by centrifugation at 14,000 rpm for 5 minutes, and the process of decanting the supernatant, adding distilled water, redispersing and centrifuging was repeated three times to wash the PC microspheres. The particles of the washed microspheres were freeze-dried to obtain a powder of microspheres.

Cy7.5-HSA-PC (without P-188), which is PC microspheres loaded with Cy7.5-HSA complex, was prepared. 1.18 mg of Cy7.5 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PC was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Cy7.5-HSA-loaded PC polymer microspheres were obtained by centrifugation at 14,000 rpm for 5 minutes, and the process of decanting the supernatant, adding distilled water, redispersing and centrifuging was repeated three times to wash the PC microspheres. The particles of the washed microspheres were freeze-dried to obtain a powder of microspheres.

To evaluate the content and loading rate of Cy7.5 in the prepared microspheres, the microspheres were dissolved and the concentration of Cy7.5 in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the Cy7.5-human serum albumin complex, the same concentration of Cy7.5 complex solution and dispersion of microparticles were prepared. 0.70 mg of Cy7.5 powder was dissolved in a mixed solution of 0.214 mL of human serum albumin solution (20% concentration) and 0.786 mL of distilled water, and then diluted with distilled water to prepare 0.3 mL of Cy7.5-human serum albumin complex (Cy7-HSA) at a concentration of 30 µM. Using the information on the content of Cy7.5 in PC microspheres evaluated by the quantification, a dispersion of microspheres was prepared by adding Cy7.5 to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader.

When preparing PMMA microspheres, it was confirmed that the loading rate of Cy7.5 in Cy7.5-PMMA (without P-188) microspheres prepared by loading Cy7.5 was 2.08%, and the content of Cy7.5 in 1 mg of microspheres was 20.2 ng. It was confirmed that the loading rate of Cy7.5 in Cy7.5-HSA-PMMA (1.0% P-188) microspheres prepared by loading Cy7.5-HSA and adding 1.0% poloxamer 188 based on the first emulsion was 11.8%, and the content of Cy7.5 in 1 mg of microspheres was 108.6 ng, and thus, they were loaded with a higher content of Cy7.5 than Cy7.5-PMMA (without P-188) microspheres.

As a result of analyzing scanning electron microscopy images of Cy7.5-PMMA (without P-188) and Cy7.5-HSA-PMMA (without P-188) microspheres, it was confirmed that each of the microspheres was a spherical particle with an average particle size of 58.3 ± 19.0 µm and 55.2 ± 19.5 um, and had an appropriate size to avoid the phagocytosis of macrophages ((a) and (c) in Figure 43). In addition, as a result of observing the scanning electron microscopy cross-section of each of the microspheres ((b) and (d) in Figure 43), it was confirmed that since the inside of the PMMA microspheres contained several isolated small spherical spaces, elution of the loaded fluorescent dye could be prevented, so that they were a very suitable form for long-term fluorescent dye marking.

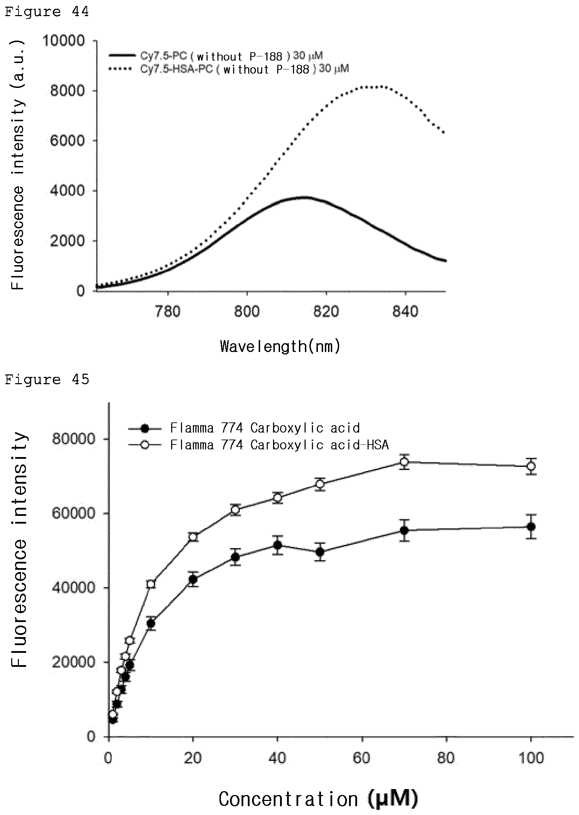

In the fluorescence spectrum of the dispersion of Cy7.5-HSA-PC (without P-188) microspheres in Figure 44, a fluorescence signal 2.2 times higher than Cy7.5-PC (without P-188) could be confirmed. From these results, it can be seen that a much higher fluorescence signal may be obtained when Cy7.5 as a near-infrared fluorescent dye is loaded in the form of a Cy7.5-HSA complex than when loaded alone in microspheres based on PC polymer.

### Example 11: Preparation and analysis of Flamm774-human serum albumin complex-loaded microspheres

### 11-1: Comparison of fluorescence signals depending on the concentration of Flamma774 solution and Flamma774-HSA complex

0.60 mg of Flamma774 carboxylic acid (Flamma774, Bioacts) as a near-infrared fluorescent dye was dissolved in 1 mL of distilled water, and then diluted with distilled water to prepare Flamma774 solutions at concentrations of 100, 70, 50, 40, 30, 20, 10, 5, 4, 3, 2 and 1 µM. For Flamma774-human serum albumin (HSA, SK Plasma) complex for comparison of fluorescence signals with Flamma774 solution, 0.60 mg of Flamma774 powder was dissolved in a mixed solution of 0.214 mL of human serum albumin solution (20% concentration) and 0.786 mL of distilled water, and then diluted with distilled water to prepare Flamma774-human serum albumin complexes (Flamma774-HSA) at concentrations of 100, 70, 50, 40, 30, 20, 10, 5, 4, 3, 2 and 1 µM. To compare fluorescence spectra, 200 µL of each prepared solution was aliquoted and placed in a 96-well plate, and the fluorescence spectrum (λₑₓ = 730 nm, λₑₘ = 812 nm (Flamma774), λₑₘ = 818 nm (Flamma774-HSA)) was measured using a multi-function microplate reader (SPARK, Tecan Trading AG, Zurich, Switzerland). Flamma774 fluorescent dye showed an emission peak at 812 nm, but after forming a complex with HAS, it showed an emission peak at 818 nm.

As shown in Figure 45, which compares the fluorescence intensity of the Flamma774 solution and the Flamma774-HSA complex solution at a concentration of 1-100 µM, it could be confirmed that the Flamma774-HSA solution generated a higher fluorescence signal than the Flamma774 solution in the entire concentration range, and it was confirmed that the Flamma774-HSA solution generated a fluorescence signal that was 1.3 times higher than the Flamma774 solution at a concentration of 30 µM and 1.3 times higher at a concentration of 100 µM, which is the maximum concentration measured.

### 11-2: Preparation and analysis of microspheres loaded with Flamma774 and Flamma774-HSA complex

Flamma774-PMMA (without P-188), which is PMMA microspheres loaded with Flamma774 as a near-infrared fluorescent dye, was prepared. W1 solution was prepared by dissolving 1.01 mg of Flamma774 in 1.691 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Flamma774-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

Flamma774-HSA-PMMA (without P-188), which is PMMA microspheres loaded with Flamma774-HSA complex, was prepared. 1.01 mg of Flamma774 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Flamma 77 4-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

Flamma774-HSA-PMMA (P-188 1.0%), which is PMMA microspheres comprising poloxamer 188 at a concentration of 0.1% in the first emulsion and loaded with Flamma774-HSA complex as a near-infrared fluorescent dye, was prepared. 1.01 mg of Flamma774 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA and 186 mg of poloxamer 188 (P-188) were added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Flamma 77 4-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To evaluate the content and loading rate of Flamma774 in the prepared microspheres, the microspheres were dissolved and the concentration of Flamma774 in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the Flmma774-human serum albumin complex, the same concentration of Flamma774 complex solution and dispersion of microparticles were prepared. 0.60 mg of Flamma774 powder was dissolved in a mixed solution of 0.214 mL of human serum albumin solution (20% concentration) and 0.786 mL of distilled water, and then diluted with distilled water to prepare 0.3 mL of Flamma774-human serum albumin complex (Flmma774-HSA) at a concentration of 30 µM. Using the information on the content of Flamma774 in PMMA microspheres evaluated by the quantification, a dispersion of microspheres was prepared by adding Flamma774 to 0.3 mL of distilled water so that the concentration was 30 µM. To compare fluorescence spectra, 200 µL, of each of the prepared solution and the dispersion of microspheres was aliquoted and placed in a 96-well plate, and the fluorescence spectrum (λₑₓ = 730 nm) was measured using a multi-function microplate reader.

When preparing PMMA microspheres, it was confirmed that the loading rate of Flamma774 in Flamma774-PMMA (without P-188) microspheres prepared by loading Flamma774 was 5.08%, and the content of Flamma774 in 1 mg of microspheres was 42.3 ng. It was confirmed that the loading rates of Flamma774 in Flamma774-HSA-PMMA (without P-188) prepared by loading Flamma774-HSA and Flamma774-HSA-PMMA (1.0% P-188) microspheres prepared by adding 1.0% poloxamer 188 based on the first emulsion were 23.3% and 21.6%, and the contents of Flamma774 in 1 mg of microspheres were 183.0 ng and 148.6 ng, and thus, they were loaded with a higher content of Flamma774 than Flamma774-PMMA (without P-188) microspheres loaded with Flamma774 alone.

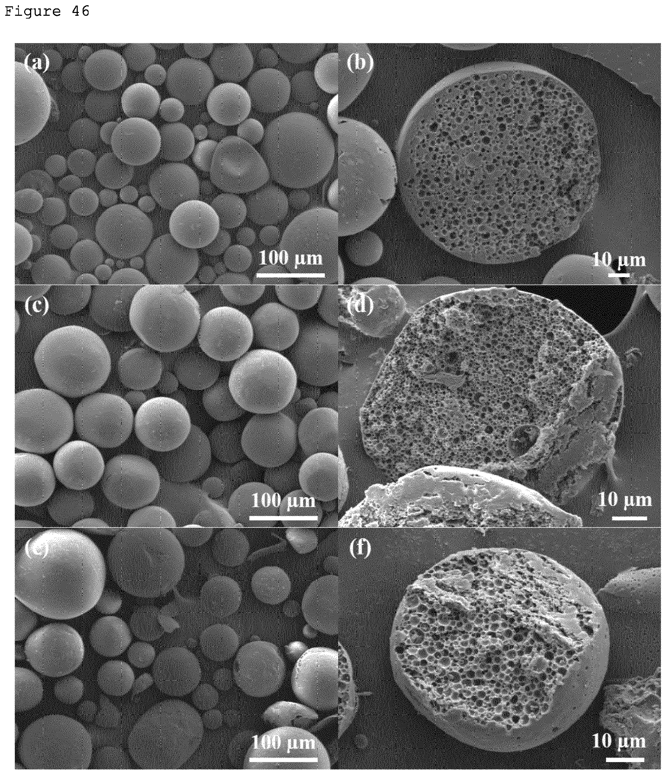

As a result of analyzing scanning electron microscopy images of Flamma774-PMMA (without P-188), Flamma774-HSA-PMMA (without P-188) and Flamma774-HSA-PMMA (1.0% P-188) microspheres, it was confirmed that each of the microspheres was a spherical particle with an average particle size of 56.8 ± 19.2 µm, 62.5 ± 19.3 µm and 55.8 ± 19.3 µm, and had an appropriate size to avoid the phagocytosis of macrophages ((a), (c) and (e) in Figure 46). In addition, as a result of analyzing the scanning electron microscopy cross-section of each of the microspheres ((b), (d) and (f) in Figure 46), it was confirmed that since the inside of the PMMA microspheres contained several isolated small spherical spaces, elution of the loaded fluorescent dye could be prevented, so that they were a very suitable form for long-term fluorescent dye marking.

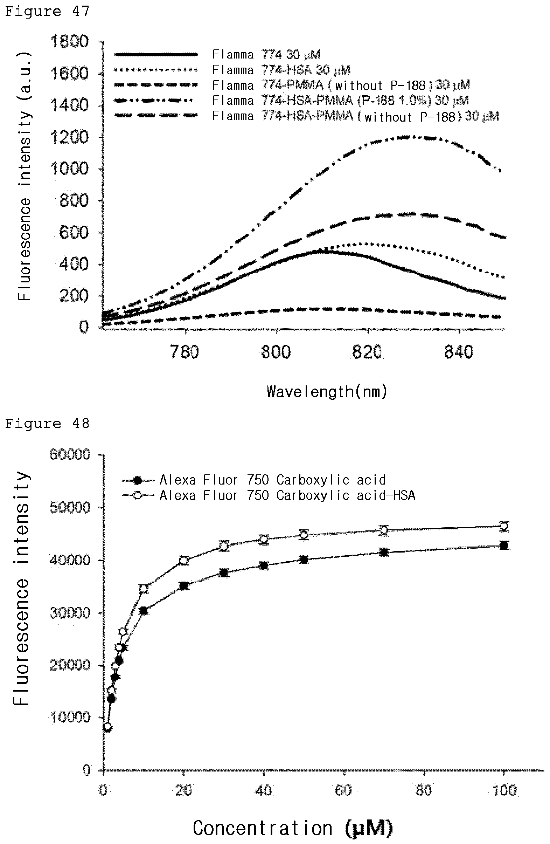

In the fluorescence spectrum of the dispersion of Flamma774-PMMA (without P-188) microspheres in Figure 47, a fluorescence signal 4.5 times lower than that of Flamma774-HSA could be confirmed, and in the fluorescence spectra of the dispersions of Flamma774-HSA-PMMA (without P-188) and Flamma774-HSA-PMMA (1.0% P-188) microspheres, fluorescence signals that were 1.4 and 2.3 times higher than that of Flamma774-HSA, respectively, and 6.2 and 10.4 times higher than that of Flamma774-PMMA (without P-188) could be confirmed. From these results, it can be seen that a much higher fluorescence signal may be obtained when Flamma774 as a near-infrared fluorescent dye is also loaded in the form of a Flamma774-HSA complex than when loaded alone.

### Example 12: Preparation and analysis of Alexa750-human serum albumin complex-loaded microspheres

### 12-1: Comparison of fluorescence signals depending on the concentration of Alexa750 solution and Alexa750-HSA complex

0.77 mg of Alexa fluor^{™} 750 carboxylic acid, tris(triethylammonium) salt (Alexa750, Thermo Fisher Scientific) as a near-infrared fluorescent dye was dissolved in 1 mL of distilled water, and then diluted with distilled water to prepare Alexa750 solutions at concentrations of 100, 70, 50, 40, 30, 20, 10, 5, 4, 3, 2 and 1 µM. For Alexa750-human serum albumin (HSA, SK Plasma) complex for comparison of fluorescence signals with Alexa750 solution, 0.77 mg of Alexa750 powder was dissolved in a mixed solution of 0.214 mL of human serum albumin solution (20% concentration) and 0.786 mL of distilled water, and then diluted with distilled water to prepare Alexa750-human serum albumin complexes (Alexa750-HSA) at concentrations of 100, 70, 50, 40, 30, 20, 10, 5, 4, 3, 2 and 1 µM. To compare fluorescence spectra, 200 µL of each prepared solution was aliquoted and placed in a 96-well plate, and the fluorescence spectrum (λₑₓ = 730 nm, λ ₑₘ= 776 nm) was measured using a multi-function microplate reader (SPARK, Tecan Trading AG, Zurich, Switzerland).

As shown in Figure 48, which compares the fluorescence intensity of the Alexa750 solution and the Alexa750-HSA complex solution at a concentration of 1-100 µM, it could be confirmed that the Alexa750-HSA solution generated a higher fluorescence signal than the Alexa750 solution in the entire concentration range, and it was confirmed that the Alexa750-HSA solution generated a fluorescence signal that was 1.1 times higher than the Alexa750 solution at a concentration of 30 µM and 1.1 times higher at a concentration of 100 µM, which is the maximum concentration measured.

### 12-2: Preparation and analysis of microspheres loaded with Alexa750 and Alexa750-HSA complex

Alexa750-PMMA (without P-188), which is PMMA microspheres loaded with Alexa750 as a near-infrared fluorescent dye, was prepared. W1 solution was prepared by dissolving 1.31 mg of Alexa750 in 1.691 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Alexa750-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

Alexa750-HSA-PMMA (without P-188), which is PMMA microspheres loaded with Alexa750-HSA complex as a near-infrared fluorescent dye, was prepared. 1.31 mg of Alexa750 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Alexa750-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

Alexa750-HSA-PMMA (P-188 1.0%), which is PMMA microspheres comprising poloxamer 188 at a concentration of 0.1% in the first emulsion and loaded with Alexa750-HSA complex as a near-infrared fluorescent dye, was prepared. 1.31 mg of Alexa750 was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA and 186 mg of poloxamer 188 (P-188) were added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of Alexa750-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To evaluate the content and loading rate of Alexa750 in the prepared microspheres, the microspheres were dissolved and the concentration of Alexa750 in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the Alexa750-human serum albumin complex, the same concentration of Alexa750 complex solution and dispersion of microparticles were prepared. 0.77 mg of Alexa750 powder was dissolved in a mixed solution of 0.214 mL of human serum albumin solution (20% concentration) and 0.786 mL of distilled water, and then diluted with distilled water to prepare 0.3 mL of Alexa750-human serum albumin complex (Alexa750-HSA) at a concentration of 30 µM. Using the information on the content of Alexa750 in PMMA microspheres evaluated by the quantification, a dispersion of microspheres was prepared by adding Alexa750 to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum λₑₓ = 730 nm) was measured using a multi-function microplate reader.

When preparing PMMA microspheres, it was confirmed that the loading rate of Alexa750 in Alexa750-PMMA (without P-188) microspheres prepared by loading Alexa750 was 14.1%, and the content of Alexa750 in 1 mg of microspheres was 151.8 ng. It was confirmed that the loading rate of Alexa750 in Alexa750-HSA-PMMA (without P-188) microspheres prepared by loading Alexa750-HSA was 9.61%, and the content of Alexa750 in 1 mg of microspheres was 97.7 ng. It was confirmed that the loading rate of Alexa750 in Alexa750-HSA-PMMA (1.0% P-188) microspheres prepared by adding 1.0% poloxamer 188 based on the first emulsion was 21.2%, and the content of Alexa750 in 1 mg of microspheres was 188.1 ng, and thus, they were loaded with a higher content of Alexa750 than the other two microspheres.

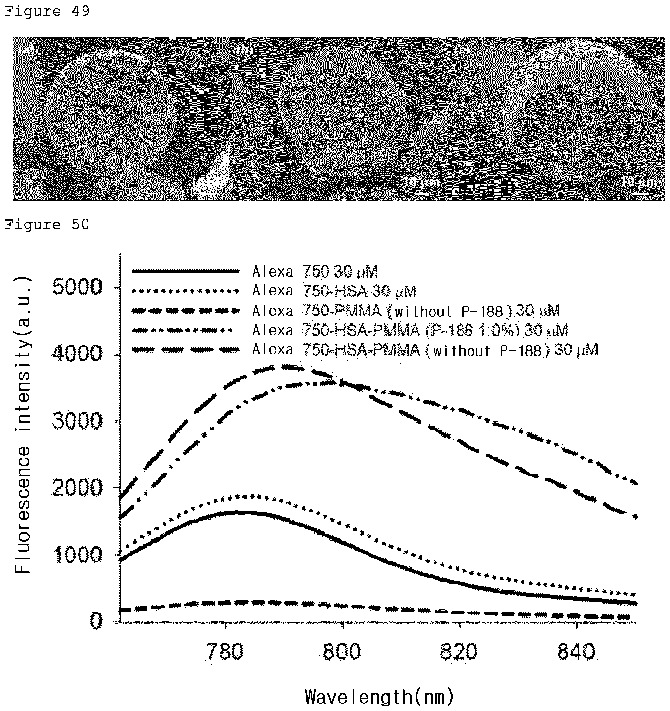

It was confirmed that Alexa750-PMMA (without P-188), Alexa750-HSA-PMMA (without P-188) and Alexa750-HSA-PMMA (1.0% P-188) microspheres were spherical particles with average particle sizes of 53.0 ± 15.8 µm, 54.6 ± 20.3 µm and 57.7 ± 19.6 um, and had an appropriate size to avoid the phagocytosis of macrophages. As a result of evaluating the scanning electron microscopy cross-section of each of the microspheres (Figure 49), it was confirmed that since the inside of the PMMA microspheres contained several isolated small spherical spaces, elution of the loaded fluorescent dye could be prevented, so that they were a very suitable form for long-term fluorescent dye marking.

In the fluorescence spectrum of the dispersion of Alexa750-PMMA (without P-188) microspheres in Figure 50, a fluorescence signal 6.5 times lower than that of Alexa750-HSA could be confirmed, and in the fluorescence spectra of the dispersions of Alexa750-HSA-PMMA (without P-188) and Alexa750-HSA-PMMA (1.0% P-188) microspheres, fluorescence signals that were 2.0 and 1.9 times higher than that of Alexa750-HSA, respectively, and 13 and 12 times higher than that of Alexa750-PMMA (without P-188) could be confirmed. From these results, it can be seen that a much higher fluorescence signal may be obtained when Alexa750 as a near-infrared fluorescent dye is also loaded in the form of a Alexa750-HSA complex than when loaded alone.

### Example 13: Preparation and analysis of IRDye 800CW-human serum albumin complex-loaded microspheres

### 13-1: Comparison of fluorescence signals depending on the concentration of IRDye 800CW solution and IRDye 800CW-HSA complex

0.70 mg of IRDye^{®} 800CW carboxylate (IRDye 800CW, LI-COR, Inc.) as a near-infrared fluorescent dye was dissolved in 1 mL of distilled water, and then diluted with distilled water to prepare Alexa750 solutions at concentrations of 100, 70, 50, 40, 30, 20, 10, 5, 4, 3, 2 and 1 µM. For IRDye 800CW-human serum albumin (HSA, SK Plasma) complex for comparison of fluorescence signals with IRDye 800CW solution, 0.70 mg of IRDye 800CW powder was dissolved in a mixed solution of 0.214 mL of human serum albumin solution (20% concentration) and 0.786 mL of distilled water, and then diluted with distilled water to prepare IRDye 800CW-human serum albumin complexes (IRDye 800CW-HSA) at concentrations of 100, 70, 50, 40, 30, 20, 10, 5, 4, 3, 2 and 1 µM. To compare fluorescence spectra, 200 µL of each prepared solution was aliquoted and placed in a 96-well plate, and the fluorescence spectrum (λₑₓ = 730 nm, λₑₘ = 806 nm (IRDye 800CW), λₑₘ = 810 nm (IRDye 800CW-HSA)) was measured using a multi-function microplate reader (SPARK, Tecan Trading AG, Zurich, Switzerland). IRDye 800CW fluorescent dye showed an emission peak at 806 nm, but after forming a complex with HAS, it showed an emission peak at 810 nm.

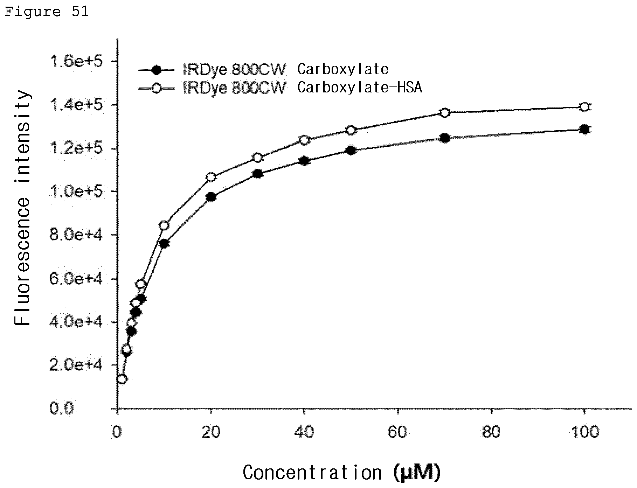

As shown in Figure 51, which compares the fluorescence intensity of the IRDye 800CW solution and the IRDye 800CW-HSA complex solution at a concentration of 1-100 µM, it could be confirmed that the IRDye 800CW-HSA solution generated a higher fluorescence signal than the IRDye 800CW solution at a concentration of 3 µM or more, and it was confirmed that the IRDye 800CW-HSA solution generated a fluorescence signal that was 1.1 times higher than the IRDye 800CW solution at a concentration of 30 µM and 1.1 times higher at a concentration of 100 µM, which is the maximum concentration measured.

### 13-2: Preparation and analysis of microspheres loaded with IRDye 800CW and IRDye 800CW-HSA complex

IRDye 800CW-PMMA (without P-188), which is PMMA microspheres loaded with IRDye 800CW as a near-infrared fluorescent dye, was prepared. W1 solution was prepared by dissolving 1.19 mg of IRDye 800CW in 1.691 mL of distilled water. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of IRDye 800CW-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

IRDye 800CW-HSA-PMMA (without P-188), which is PMMA microspheres loaded with IRDye 800CW-HSA complex as a near-infrared fluorescent dye, was prepared. 1.19 mg of IRDye 800CW was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA was added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of IRDye 800CW-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

IRDye 800CW-HSA-PMMA (P-188 1.0%), which is PMMA microspheres comprising poloxamer 188 at a concentration of 0.1% in the first emulsion and loaded with IRDye 800CW-HSA complex as a near-infrared fluorescent dye, was prepared. 1.19 mg of IRDye 800CW was dissolved in 0.363 mL of human serum albumin (HSA) (20% concentration, 72.553 mg) and 1.328 mL of distilled water to prepare W1 solution. 3.6 g of poly(vinyl alcohol) was added to 180 mL of distilled water, and then dissolved while stirring at 600 rpm under a condition of 85°C and cooled to room temperature to prepare W2 solution. 1.217 g of PMMA and 186 mg of poloxamer 188 (P-188) were added to 16.909 mL of methylene chloride and then dissolved to prepare an oil solution. The prepared W1 solution was added to the oil solution, and then sonicated in a probe-type ultrasonic device for 3 minutes (repeated 1-minute sonication + 30-second rest) to prepare a first emulsion (water-in-oil). While stirring the prepared W2 solution at 1000 rpm using a mechanical stirrer, the prepared first emulsion solution was added thereto at a rate of 10 mL/min using a syringe pump to prepare a second emulsion (water-in-oil-in-water). The prepared emulsion was stirred at 1000 rpm for 10 minutes, and then stirred at 280 rpm for 21 hours using a magnetic stirrer under a temperature condition of 40°C to remove the methylene chloride solvent. Afterwards, the particles of IRDye 800CW-HSA-loaded PMMA polymer microspheres were obtained by centrifugation at 14,000 rpm for 5 minutes, and the process of decanting the supernatant, adding distilled water, redispersing and centrifuging was repeated three times to wash the PMMA microspheres. The particles of the washed microspheres were freeze-dried to obtain a powder of microspheres.

To evaluate the content and loading rate of IRDye 800CW in the prepared microspheres, the microspheres were dissolved and the concentration of IRDye 800CW in the solution was quantified using an ultraviolet/visible light absorption spectrometer.

To analyze the particle shape and size of the synthesized microspheres, scanning electron microscope analysis was performed. For cross-sectional analysis of the prepared microspheres, the microspheres were cut with a razor blade. The powder of the microspheres was attached to a carbon tape attached to the sample holder of a scanning electron microscope, and Pt/Pd was coated on the surface through sputtering for 60 seconds, and then the scanning electron microscope images were analyzed at an acceleration voltage of 5 kV. The particle size was calculated as the average value by randomly measuring the size of 100 particles from the acquired images.

To compare the fluorescence signals of the prepared microspheres and the IRDye 800CW-human serum albumin complex, the same concentration of Alexa750 complex solution and dispersion of microparticles were prepared. 0.70 mg of IRDye 800CW powder was dissolved in a mixed solution of 0.214 mL of human serum albumin solution (20% concentration) and 0.786 mL of distilled water, and then diluted with distilled water to prepare 0.3 mL of IRDye 800CW-human serum albumin complex (IRDye 800CW-HSA) at a concentration of 30 µM. Using the information on the content of IRDye 800CW in PMMA microspheres evaluated by the quantification, a dispersion of microspheres was prepared by adding IRDye 800CW to 0.3 mL of distilled water so that the concentration was 30 µM. To compare fluorescence spectra, 200 µL of each of the prepared solution and the dispersion of microspheres was aliquoted and placed in a 96-well plate, and the fluorescence spectrum (λₑₓ = 730 nm) was measured using a multi-function microplate reader.

When preparing PMMA microspheres, it was confirmed that the loading rate of IRDye 800CW in IRDye 800CW-PMMA (without P-188) microspheres prepared by loading IRDye 800CW was 10.7%, and the content of IRDye 800CW in 1 mg of microspheres was 104.4 ng. It was confirmed that the loading rates of IRDye 800CW in IRDye 800CW-HSA-PMMA (without P-188) microspheres prepared by loading IRDye 800CW-HSA and IRDye 800CW-HSA-PMMA (1.0% P-188) microspheres prepared by adding 1.0% poloxamer 188 based on the first emulsion were 19.5% and 19.0%, and the contents of IRDye 800CW in 1 mg of microspheres were 179.6 ng and 152.8 ng, and thus, they were loaded with a higher content of IRDye 800CW than IRDye 800CW-PMMA (without P-188) microspheres.

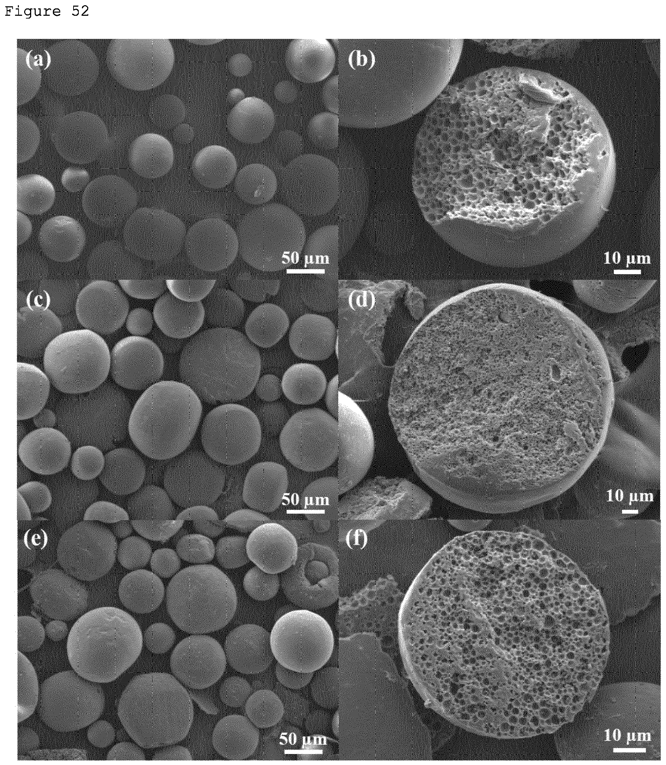

As a result of analyzing scanning electron microscopy images of IRDye 800CW-PMMA (without P-188), IRDye 800CW-HSA-PMMA (without P-188) and IRDye 800CW-HSA-PMMA (1.0% P-188) microspheres, it was confirmed that each of the microspheres was a spherical particle with an average particle size of 55.8 ± 19.7 um, 66.6 ± 13.8 µm and 59.8 ± 17.3 µm, and had an appropriate size to avoid the phagocytosis of macrophages ((a), (c) and (e) in Figure 52). In addition, as a result of analyzing the scanning electron microscopy cross-section of each of the microspheres ((b), (d) and (f) in Figure 52), it was confirmed that since the inside of the PMMA microspheres contained several isolated small spherical spaces, elution of the loaded fluorescent dye could be prevented, so that they were a very suitable form for long-term fluorescent dye marking.

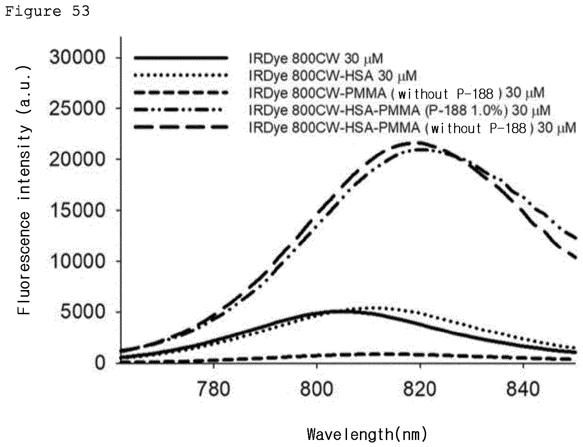

In the fluorescence spectrum of the dispersion of IRDye 800CW-PMMA (without P-188) microspheres in Figure 53, a fluorescence signal 6.2 times lower than that of IRDye 800CW-HSA could be confirmed, and in the fluorescence spectra of the dispersions of IRDye 800CW-HSA-PMMA (without P-188) and IRDye 800CW-HSA-PMMA (1.0% P-188) microspheres, fluorescence signals that were 4.0 and 3.9 times higher than that of IRDye 800CW-HSA, respectively, and 25 and 24 times higher than that of IRDye 800CW-PMMA (without P-188) could be confirmed. From these results, it can be seen that a much higher fluorescence signal may be obtained when IRDye 800CW as a near-infrared fluorescent dye is also loaded in the form of a IRDye 800CW-HSA complex than when loaded alone.

### Example 14: Preparation of a dispersion composition of PLGA microspheres and preparation and analysis of a marker in the form of a pellet containing microspheres

Microspheres prepared using PLGA polymer as a representative example of a biodegradable polymer were dispersed in various other surfactants or biocompatible polymer materials, and an aqueous dispersion composition solution for injection was prepared. The microspheres used in this example are ICG-HSA-PLGA (P-188 0.5%). For dispersion of microspheres, a solution of 0.5% sodium methylcellulose (Sigma-Aldrich)/0.1% Tween 80 (Sigma-Aldrich) dissolved in physiological saline for injection, 1% sodium alginate (Kimica), 1% sodium hyaluronate (MW 1M) (Lifecore biomedical), 2% soidum methylcellulose (Sigma-Aldrich), 2% poly(vinyl alcohol) (Sigma-Aldrich), 0.1% collagen (collagen from calf skin, Sigma-Aldrich), 5% gelatin (gelatin from porcine, Sigma-Aldrich), 10% Tween 80 (Sigma-Aldrich), 40% poloxamer 188 (BASF), 5% polyvinyl pyrrolidone (Sigma-Aldrich) solution were prepared, and then the microspheres were dispersed in each solution so that the concentration of indocyanine green in the ICG-HSA-PLGA (P-188 0.5%) microspheres was 30 µM.

To prepare a formulation for marking lesions in solid form, markers in the form of dried pellets were prepared. For this purpose, the microspheres dispersed in 1% sodium hyaluronate and 5% aqueous gelatin solution were transferred to a cylindrical frame, and then rapidly cooled using liquid nitrogen and freeze-dried to prepare a fluorescence marker in the form of a solid pellet.

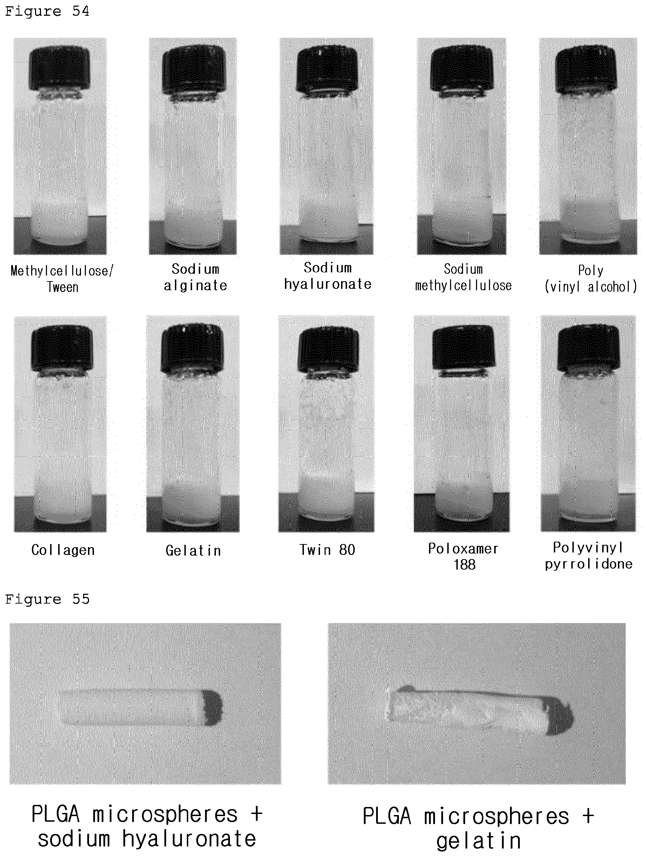

The results of preparing a composition in which microspheres were dispersed in various different surfactants and biocompatible polymer materials are shown in Figure 54. It was confirmed that ICG-HSA-PLGA (P-188 0.5%) microspheres were evenly dispersed in all composition solutions used.

In addition, according to Figure 55, it can be confirmed that a fluorescence marker in the form of a solid pellet prepared using microspheres dispersed in a 1% sodium hyaluronate and 5% gelatin solution may be prepared.

### Example 15: Preparation of a dispersion composition of PMMA microspheres and preparation and analysis of a marker in the form of a pellet containing microspheres

Microspheres prepared using PMMA polymer as a representative example of a non-biodegradable polymer were dispersed in various other surfactants or biocompatible polymer materials, and an aqueous dispersion composition solution for injection was prepared. The microspheres used in this example are ICG-HSA-PMMA (P-188 1.0%) microspheres.

For dispersion of microspheres, a solution of 0.5% sodium methylcellulose (Sigma-Aldrich)/0.1% Tween 80 (Sigma-Aldrich) dissolved in physiological saline for injection, 1% sodium alginate, 1% sodium hyaluronate, 2% soidum methylcellulose, 2% poly(vinyl alcohol), 0.1% collagen, 5% gelatin, 10% Tween 80, 40% poloxamer 188, 5% polyvinyl pyrrolidone solution were prepared, and then the microspheres were dispersed in each solution so that the concentration of indocyanine green in the ICG-HSA-PMMA (P-188 1.0%) microspheres was 30 µM.

To prepare a formulation for marking lesions in solid form, markers in the form of dried pellets were prepared. For this purpose, the microspheres dispersed in an aqueous solution of 1% sodium alginate and 1% sodium hyaluronate, 2% soidum methylcellulose, 2% poly(vinyl alcohol), 5% gelatin, 40% poloxamer 188, 5% polyvinyl pyrrolidone were transferred to a cylindrical frame, and then rapidly cooled using liquid nitrogen and freeze-dried to prepare a fluorescence marker in the form of a solid pellet.

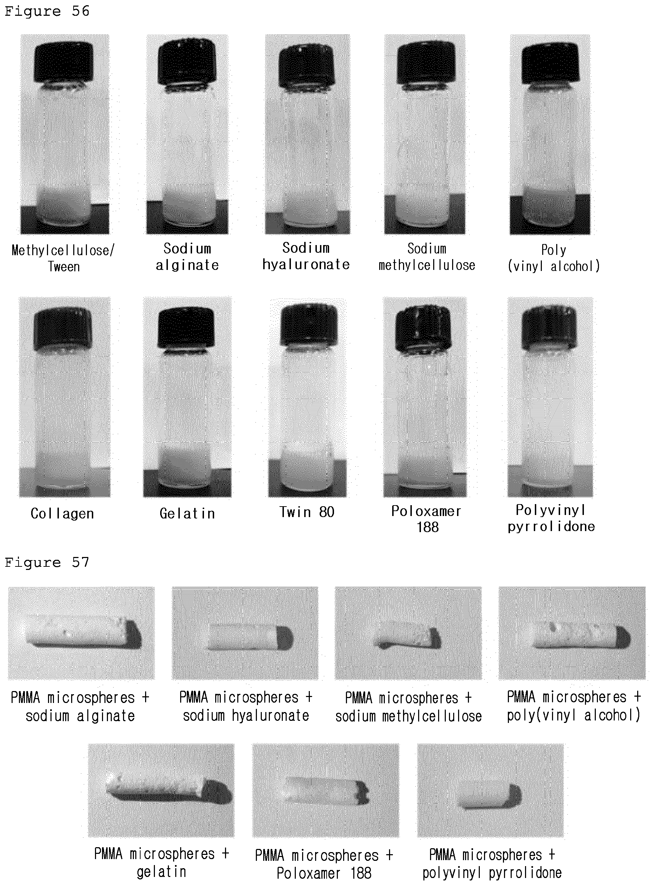

As a result of preparing a composition in which ICG-HSA-PMMA (P-188 1.0%) microspheres were dispersed in various other surfactants and biocompatible polymer materials, as shown in Figure 56, it was confirmed that ICG-HSA-PMMA (P-188 1.0%) microspheres were evenly dispersed in all composition solutions used.

In addition, according to the results in Figure 57, it could be confirmed that a fluorescence marker in the form of a solid pellet prepared using microspheres dispersed in a solution of 1% sodium alginate and 1% sodium hyaluronate, 2% soidum methylcellulose, 2% poly(vinyl alcohol), 5% gelatin, 40% poloxamer 188, 5% polyvinyl pyrrolidone could be prepared.

### Example 16: Preparation and analysis of near-infrared fluorescence marker in the form of a metal clip coated with microspheres

This example was intended to show that it is possible to prepare fluorescent markers using metallic and non-metallic materials by coating the surfaces of metallic and non-metallic materials with fluorescent dye-loaded microspheres. For this purpose, metal clips coated with fluorescent dye-loaded microspheres were prepared by dispersing IRDye 800CW-HSA-PMMA (without P-188) microspheres, which are microspheres prepared using PMMA polymer, in a latex solution and then coating them on the clip surface and drying.

To prepare a coating solution in which IRDye 800CW-HSA-PMMA (without P-188) microspheres were dispersed, the coating solution was prepared by dispersing the microspheres in a latex solution (36% acrylonitrile-butadinene copolymer, 64% water) so that the concentration of the microspheres was 135.03 mg/mL. Afterwards, 2 µL of the coating solution was evenly coated on both surfaces of the metal clip. The solution was coated on the surface of the metal clip and dried at room temperature for 24 hours, and then heat treated at 100°C for 30 minutes to prepare a metal clip capable of functioning as a fluorescent marker coated with IRDye 800CW-HSA-PMMA (without P-188) microspheres.

To evaluate the efficacy of the IRDye 800CW-HSA-PMMA (without P-188) microspheres-coated metal clip as a fluorescent marker, one side of the IRDye 800CW-HSA-PMMA (without P-188) microspheres-coated fluorescent clip was cut, and fluorescence images were taken with the coated side facing upward (λₑₓ = 780/20 nm, λₑₘ = 845/40 nm) .

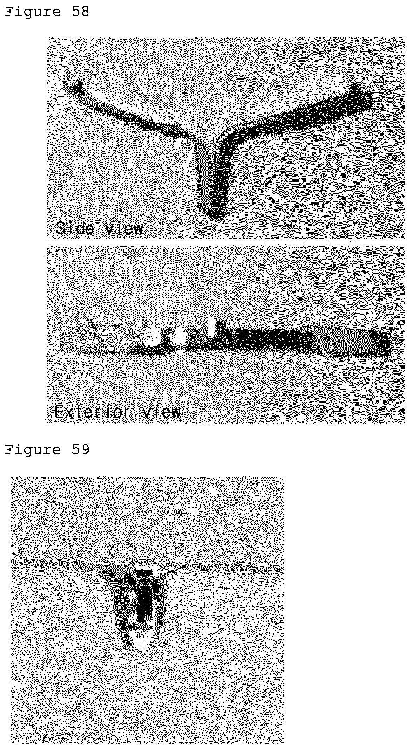

As shown in the photographs in Figure 58, it could be confirmed that the microspheres were evenly coated on the surface of the metal clip.

It was confirmed that the ROI (region of interest) value analyzed from the fluorescence image of the microspheres-coated metal clip was 4.92 × 10⁵, and a stronger fluorescence signal was generated compared to the ICG-HAS complex at the same concentration. From the results of Figures 58 and 59, it could be confirmed that various types of fluorescent markers may be prepared by coating near-infrared fluorescent dye-loaded microspheres on metal clips, and the like.

From the above description, those of ordinary skill in the technical art to which the present invention pertains will understand that the present invention may be implemented in other specific forms without changing the technical spirit or essential characteristics thereof. In this regard, it should be understood that the examples described above are illustrative and not restrictive in all respects. It should be construed that all changes or modifications derived from the meaning and scope of the claims to be described later rather than the detailed description and their equivalent concepts are included in the scope of the present invention.

## Claims

1. Near-infrared fluorescent dye-loaded polymer microspheres for marking lesions, wherein the near-infrared fluorescent dye forms a complex with human serum albumin or cyclodextrin.

2. The polymer microspheres according to claim 1, wherein the near-infrared fluorescent dye is any one selected from indocyanine green, IRDye 800CW carboxylate (IRDye 800CW), Alexa Fluor 647, Alexa Fluor 660, Alexa Fluor 680, Alexa Fluor 700, Alexa Fluor 750, Alexa Fluor 780, Flamma 749, Flamma 774, Flamma 800, FSD Fluor^{™} 647, FSD Fluor^{™} 680, FSD Fluor^{™} 750, FSD Fluor^{™} 800, sulfo-cyanine 5 carboxylic acid (Cy5), sulfo-cyanine 5.5 carboxylic acid (Cy5.5), sulfo-cyanine 7 carboxylic acid (Cy7) or sulfo-cyanine 7.5 carboxylic acid (Cy7.5).

3. The polymer microspheres according to claim 1, wherein the polymer is poly(lactide-co-glycolide) (PLGA), poly(DL-lactide-co-glycolide) (PDLGA), poly(glycolic acid) (PGA), poly(lactide) (PLA), poly(hydroxybutyrate), polycaprolactone (PCL), polydioxanone (PDO), poly(amino acid), polyanhydride, polyorthoester or polyphosphazene.

4. The polymer microspheres according to claim 3, comprising a block copolymer of the polymer and poly(ethylene oxide) (PEG).

5. The polymer microspheres according to claim 1, wherein the polymer is poly(methyl methacrylate) (PMMA) or polycarbonate (PC).

6. The polymer microspheres according to claim 1, wherein the microspheres further comprise alginic acid or hyaluronic acid.

7. The polymer microspheres according to claim 1, wherein the microspheres further comprise a surfactant.

8. The polymer microspheres according to claim 7, wherein the surfactant is any one selected from polyvinyl alcohol, polyethylene glycol, Labrafil, Labrasol, medium chain triglyceride, lecitin, N-methyl pyrrolidone, polyvinyl pyrrolidone, hydropropyl methylcellulose, poloxamer or Tween.

9. The polymer microspheres according to claim 1, wherein the microspheres comprise one or more cavities within the microspheres, and the complex is loaded into the cavities.

10. The polymer microspheres according to claim 1, wherein the microspheres are for coating on the surface of a metal or non-metallic material.

11. The polymer microspheres according to claim 10, wherein the coating is made with adhesives or rubber.

12. A method for preparing polymer microspheres for marking lesions, comprising the steps of:
forming a complex of a near-infrared fluorescent dye and human serum albumin, or a near-infrared fluorescent dye and cyclodextrin; and
loading the complex into polymer microspheres.

13. The method according to claim 12, wherein a water-in-oil-in-water (W1/O/W2) emulsion method is used as a method for loading the complex into the polymer microspheres.

14. The method according to claim 12, further comprising mixing the near-infrared fluorescent dye with a hydrogel polymer to load it into the polymer microspheres.

15. A composition for marking lesions, comprising the polymer microspheres according any one of claims 1 to 11.

16. The composition according to claim 15, wherein the microspheres are mixed with methylcellulose, alginic acid, hyaluronic acid, polyvinyl alcohol, collagen, gelatin, Tween, poloxamer or polyvinyl pyrrolidone.

17. The composition according to claim 16, wherein the composition is injectable through a syringe.

18. The composition according to claim 15, wherein the composition is in the form of a solid pellet.

19. A method for marking lesions, comprising the step of injecting the composition according to claim 15 into an individual.

20. Use of the composition according to claim 15 for marking lesions.
